(19) Europäisches Patentamt
European Patent Office
Office européen des brevets

(11) Veröffentlichungsnummer: **0 107 759 B1**

(12) **EUROPÄISCHE PATENTSCHRIFT**

(45) Veröffentlichungstag der Patentschrift: **16.12.92**

(21) Anmeldenummer: **83107798.7**

(22) Anmeldetag: **08.08.83**

(51) Int. Cl.⁵: **C07C 255/45**, C07D 211/14, C07D 317/14, C07D 239/26, C07C 13/28, C07C 23/18, C07C 19/02, C07C 69/75, C09K 19/30

# (54) Cyclohexanderivate und ihre Verwendung als Komponenten Flüssigkristalliner-Dielektrika.

(30) Priorität: **26.08.82 DE 3231707**
**03.06.83 DE 3320024**

(43) Veröffentlichungstag der Anmeldung:
**09.05.84 Patentblatt 84/19**

(45) Bekanntmachung des Hinweises auf die Patenterteilung:
**16.12.92 Patentblatt 92/51**

(84) Benannte Vertragsstaaten:
**AT CH DE FR GB IT LI NL**

(56) Entgegenhaltungen:
**EP-A- 0 050 023**
**DE-A- 1 670 386**
**GB-A- 851 782**
**GB-A- 2 092 169**
**US-A- 3 413 309**
**EP-A- 0 051 738**
**DE-A- 2 929 509**
**GB-A- 2 078 389**
**GB-A- 2 111 993**
**US-A- 3 979 444**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Eidenschink, Rudolf, Dr.**
**Erlenweg 17**
**W-6110 Dieburg(DE)**
Erfinder: **Haas, Günther, Dr.**
**Saarstrasse 109**
**W-6903 Neckargemünd(DE)**
Erfinder: **Pohl, Ludwig, Dr.**
**Niebergallweg 5**
**W-6100 Darmstadt(DE)**
Erfinder: **Römer, Michael, Dr.**
**Im grossen Garten 18**
**W-6054 Rodgau 2(DE)**
Erfinder: **Scheuble, Bernhard, Dr.**
**Am Grenzweg 18**
**W-6146 Alsbach(DE)**
Erfinder: **Weber, Georg**
**Wilhelm-Leuschner-Strasse 38**
**W-6106 Erzhausen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

BULLETIN DE LA SOCIETE CHIMIOUE DE FRANCE, Nr. 7/8, 1973, Seiten 2313-2317, Abschnitt Nr. 406 J.-P. MAZALEYRAT: "Influence de la substitution géminale sur la solvatation des amines cyclohexaniques."

CHEMICAL ABSTRACTS, Band 62, Nr. 12, 7. Juni 1965, Spalte 14546f; Coolumbus, Ohio, US A.I. KAKHNIASHVILI et al.: "Condensation of phenol with 1-vinyl-1-cyclohexanol in presence of phosphoric acid, and with 1-ethyl-1-cyclohexanol in presence of 80% sulfuric acid."

CHEMICAL ABSTRACTS REGISTRY HANDBOOK, Number Section, 1976, Teil 2; Seite 2152RE, Ref.Nr. 60756-85-6; Columbus, Ohio, US

CHEMICAL ABSTRACTS, and 51 , Nr. 2, 25. Januar 1957, Spalte 1923a; Columbus, Ohio, US S.I, KHROMOV et al.: "Contact transformations of 1,1'-dimethyldicyclohexyl in the presence of platinized carbon."

MONATSHEFTE DER CHEMIE, Band 91, Nr. 6, 1960, Seiten 1043-1501 H. BUDZIKIEWICZ et al.: "Reaktionen des 2,2-Dimethylcyclohexadienons, 2. Mitt.: Umsetzuung mit Phenylmagnesiumbromid."

JOURNAL OF THE AMERICAN CHEMICAL SOCIETY, Band 71, Nr. 5, 19. Mai 1949, Seiten 1705-1709 C.H. TILFORD et al.: "Aminoesters of 1-substituted alicyclic carboxylic acids."

CHEMICAL ABSTRACTS, Band 76, Nr. 23, 4. Juni 1972, Seite 467, Ref. Nr. 1400630z; Columbus, Ohio, US A.L. MNDZHOYAN et al.: "Semisynthetic penicillins. V. (1-Alkoxyphenyl)-cyclohexyl)penicillins."

CHEMICAL ABSTRACTS; Band 72, Nr. 19, 11. Mai 1970, Seite 323, Ref. Nr. 100241j; Columbus, Ohio, US S.D. MEKHTIEV et al.: "Synthesis of aryl-substituted cyclohexyl cyanides."

JOURNAL OF MEDICINAL CHEMISTRY, Band 24, Nr. 5, 1981, Seiten 496-499 Y. ITZHAK et al.: "New analgesic drugs derived from phencyclidine."

COMPTES RENDUS HEBDOMADAIRES DES SEANCES DE L'ACADEMIE DES SCIENCES, Band 272, Nr. 18, Teil C, 3. Mai 1971, Seiten 1554-1557; Paris, FR J.-P. COIC et al.: "Synthèse d'esters de cyanhydrines en milieu anhydre."

CHEMICAL ABSTRACTS REGISTRY HANDBOOK; Number Section, 1979, Teil 2, Seite 1988RH, Ref.Nr. 71486-43-6; Columbus, Ohio, US

CHEMICAL ABSTRACTS, Band 87, Nr. 23, 5. Dezember 1977, Seite 620, Ref.Nr. 184591u; Columbus, Ohio, US A. ALBEROLA et al.: "Alkylaluminums. X. The reaction of cyclic enamines and their perchlorates with organo-aluminum compounds."

CHEMICAL ABSTRACTS, Band 85, Nr. 3, 19. Juli 1976, Seite 16, Ref. Nr. 13673b; Columbus, Ohio, US A. KALIR et al.: "1-Phenylcycloalkylamine derivatives.III."

H.-G. BOIT: "BEILSTEINS HANDBUCH DER ORGANISCHEN CHEMIE"; Viertes Ergänzungswerk, Band 5, Teil 1, 1976, Seiten 335,336; Springer Verlag, DE

JOURNAL OF THE AMERICAN CHEMICAL SOCIETY, Band 6, Nr. 12, 17. Dezember 1941, Seiten 3321, 3322 R.D. KLEENE et al.: "Position of two alicyclic hydrocarbons in the acidity series."

JOURNAL OF ORGANIC CHEMISTRY, Band 16, Nr. 2, Februar 1951, Seiten 185-191, The Williams & Wilkins Company, Baltimore, US NG.PH. BUU-HOI et al.: "Condensation of beta-naphthol with cyclohexanol and its methyl and ethyl homologs."

CHEMICAL ABSTRACTS, Band 97, Nr. 14, 4. Oktober 1982, Seite 560, Ref.Nr. 118266x; Columbus, Ohio, US & JP-A.82 21 479

CHEMICAL ABSTRACTS, Band 95, Nr. 22, 30. November 1981, Seite 567, Ref.Nr. 195221q; Columbus, Ohio, US

## Beschreibung

Die Erfindung betrifft Cyclohexanderivate der Formel I

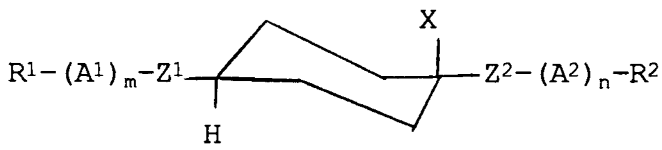

worin

| | |
|---|---|
| $R^1$ und $R^2$ | jeweils geradkettige oder höchstens einfach Alkylgruppen mit 1-10 C-Atomen, worin auch eine oder zwei $CH_2$-Gruppen durch O-Atome ersetzt sein können, F, Cl, Br, CN oder -O-COR, |
| $A^1$ und $A^2$ | jeweils unsubstituierte oder durch 1-4 F-Atome substituierte 1,4-Phenylen-, 1,4-Cyclohexylen, 1,3-Dioxan-2,5-diyl-, Piperidin-1,4-diyl-, 1,4-Bicyclo(2,2,2)-octylen- oder Pyrimidin-2,5-diyl-gruppen, |
| X | Alkyl, Alkoxy, fluoriertes Alkyl oder fluoriertes Alkoxy mit jeweils 1 - 5 C-Atomen, F, Cl, Br oder CN, |
| $Z^1$ und $Z^2$ | jeweils -CO-O-, -O-CO-, $-CH_2CH_2-$, $-OCH_2-$, $-CH_2O$-oder eine Einfachbindung, |
| R | eine Alkylgruppe mit 1 - 5 C-Atomen, |
| m | 1 oder 2 und |
| n | 0 oder 1 |

bedeuten,
wobei für m = 2 die beiden Gruppen $A^1$ gleich oder voneinander verschieden sein können, und die Gruppen $R^1$-$(A^1)_m$-$Z^1$- und -$Z^2$-$(A^2)_n$-$R^2$ in trans-Stellung zueinander stehen, während der Substituent X in cis-Stellung zu der gegenüberstehenden Gruppe steht.

Der Einfachheit halber bedeuten im folgenden "Phe" eine 1,4-Phenylengruppe, "Cy" eine 1,4-Cyclohexylengruppe, "Dio" eine 1,3-Dioxan-2,5-diylgruppe, "Bi" eine Bicyclo(2,2,2)-octylengruppe, "Pip" eine Piperidin-1,4-diylgruppe, und "Pyr" eine Pyridimin-2,5-diylgruppe, wobei diese Gruppen, insbesondere die 1,4-Phenylengruppe, unsubstituiert oder durch 1-4 Fluoratome substituiert sein können.

Ähnliche Verbindungen sind z.B. aus der EP-OS 19 665 bekannt. Die dort angegebenen Verbindungen enthalten jedoch im Gegensatz zu den vorliegenden keine 1,1,4-trisubstituierten Cyclohexanringe.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Aus den Dokumenten Bull. Soc. Chim. Fr. 1973, 2113-2317, Chem. Abstr. 51 (1957), 1923 a/b, 62, (1965), 14546 g/h, 76 (1972), 140 630z, 85 (1976), 13673 b, 87 (1977) 184 591u,
Chem. Abst. Registry Handbook Number Section, RN 60756-85-6, RN 71486-43-6, Monatshefte der Chemie 91 (1960), 1043-1051,
J. Am. Chem. Soc. 63 (1941), 3321-3322, 71 (1949) 1705-1709, J. Med. Chem. 24 (1981), 496-499, Comptes Rendus Ocad. Sci., Paris, Série C, 272 (1971), 1554-1557,
Beilsteins Handbuch der Org. Chemie, 4. Ergänzungswerk, Bd. 5, Teil 1, 1976, S. 335-335,
J. Org. Chem. 16 (1951), 185-191, US-A-341 3309, US-A-397 9444, DE-A-1 670 386 und GB-A-851 782
sind ähnliche Verbindungen bekannt, worin einer der Reste $R^1$ und $R^2$ H bedeutet und der andere dieser Reste z.B. tert.-Butyl, Methoxy, O-i-Propyl, O-$(Propyl)_2$, OEt, F, Cl oder $CH_3$ bedeutet oder gar beide Reste $R^1$ und $R^2$ H bedeuten.

Derartige Verbindungen sind als Komponenten flüssigkristalliner Dielektrika wenig geeignet.

Aus den Chem. Abstr. 72 (1970), 100 241j sind Verbindungen der Formeln

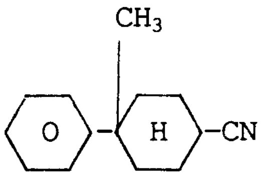

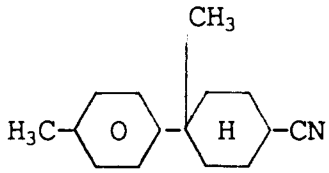

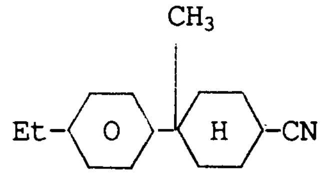

bekannt. Diese Verbindungen mit einem "innenständigem" Substituenten weisen im Vergleich zu den erfindungsgemäßen Verbindungen keine oder nur schmale Mesophasen auf.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte, sehr kleiner optischer Anisotropie und vergleichsweise niedriger Viskosität herstellbar.

Überraschenderweise zeigte sich beim Zusatz von Verbindungen der Formel I zu Mischungen mit positiver dielektrischer Anisotropie, daß selbst größere Zusätze (z.B. von 30 %) die Schwellenspannung nur unwesentlich erhöhen. Völlig unerwartet tritt gleichzeitig eine erhebliche Verbesserung der Kennliniensteilheit der Mischung auf, so daß Verbindungen des Typs I als besonders vorteilhaft geeignete Substanzen zur Herstellung von flüssigkristallinen Mischungen mit steiler Kennlinie anzusehen sind. Sie ermöglichen damit die Entwicklung hoch multiplexbarer Mischungen sehr kleiner optischer Anisotropie, mit denen eine Drehzelle insbesondere im ersten Transmissionsminimum nach Gooch-Tarry betrieben werden kann. Damit ergibt sich eine sehr kleine Beobachtungswinkel-Abhängigkeit des Kontrastes.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,
oder daß man eine Verbindung, die sonst der Formel I entspricht, aber an Stelle des Restes

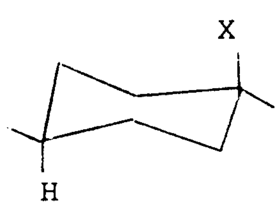

eine 1-Cyclohexen-1,4-diylgruppe enthält,

eine Verbindung der Formel HX
(worin X F, Cl, Br oder CN bedeutet) anlagert,
oder daß man zur Herstellung von Estern der Formel I (worin $R^1$ und/oder $R^2$ -O-COR bedeuten und/oder worin $Z^1$ und/oder $Z^2$ -CO-O oder -O-CO- bedeuten) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Dioxanderivaten der Formel I (worin $A^1$ und/oder $A^2$ 1,2-Dioxan-2,5-diyl bedeuten) einen entsprechenden Aldehyd mit einem entsprechenden Diol umsetzt,
oder daß man zur Herstellung von Nitrilen der Formel I (worin $R^1$ und/oder $R^2$ CN bedeuten und/oder X eine CN-Gruppe bedeutet) ein entsprechendes Carbonsäureamid dehydratisiert oder ein entsprechendes Carbonsäurehalogenid mit Sulfamid umsetzt,
oder daß man zur Herstellung von Nitrilen der Formel I (worin X CN bedeutet; worin ferner einer der Reste $Z^1$ bzw. $Z^2$ eine Einfachbindung bedeutet) ein Nitril der Formel IV,

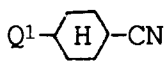

IV

worin
$Q^1$ $R^1$-$(A^1)_m$-$Z^1$-
bedeutet und
$R^1$, $A^1$, $Z^1$ und m die angegebenen Bedeutungen haben,
mit einer Verbindung der Formel V,

$Q^2$-$X^1$ V

worin
$Q^2$ $R^2$-$(A^2)_n$-
und $X^1$ Cl, Br, I, OH oder eine reaktionsfähig veresterte OH-Gruppe bedeutet und $R^2$, $A^2$ und n die angegebenen Bedeutungen haben, umsetzt,
oder daß man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ eine $-OCH_2$- oder $-CH_2O$-Gruppe ist) eine entsprechende Hydroxyverbindung verethert,
oder daß man zur Herstellung von Verbindungen der Formel I, die $CF_3$-Gruppen enthalten, eine entsprechende Carbonsäure mit $SF_4$ umsetzt,
und/oder daß man gegebenenfalls eine Chlor- oder Bromverbindung der Formel I (worin $R^1$ und/oder $R^2$ Cl oder Br bedeuten und/oder worin A durch mindestens ein Chlor- oder Bromatom substituiert ist) mit einem Cyanid umsetzt,
oder daß man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Dielektrika. Gegenstand der Erfindung sind ferner flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, $A^1$, $A^2$, $Z^1$, $Z^2$, m, n und X, die angegebene Bedeutung, sofern nicht ausdrücklich etwas anderes vermerkt ist. Der Einfachheit halber bedeutet A im folgenden

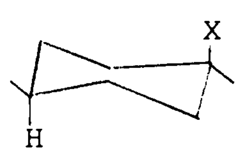

Die Verbindungen der Formel I umfassen dementsprechend Verbindungen der Teilformeln Ia (mit zwei Ringen), Ib und Ic (mit jeweils drei Ringen) sowie Id (mit vier Ringen):

$R^1$-$A^1$-$Z^1$-A-$Z^2$-$R^2$ Ia

$R^1$-$A^1$-$Z^1$-A-$Z^2$-$A^2$-$R^2$ Ib

$R^1$-$(A^1)_2$-$Z^1$-A-$Z^2$-$R^2$ Ic

$R^1$-$(A^1)_2$-$Z^1$-A-$Z^2$-$A^2$-$R^2$ Id.

Die bevorzugten Verbindungen der Teilformel Ia umfassen solche der Teilformeln Ie bis Ij:

$R^1$-Phe-$Z^1$-A-$Z^2$-$R^2$ Ie

$R^1$-Cy-$Z^1$-A-$Z^2$-$R^2$ If

$R^1$-Dio-$Z^1$-A-$Z^2$-$R^2$ Ig

$R^1$-Pip-$Z^1$-A-$Z^2$-$R^2$ Ih

$R^1$-Bic-$Z^1$-A-$Z^2$-$R^2$ Ii

$R^1$-Pyr-$Z^1$-A-$Z^2$-$R^2$ Ij

Darunter sind diejenigen der Formeln Ie, If und Ig besonders bevorzugt.

Von den Verbindungen der Teilformeln Ib, Ic und Id sind diejenigen der Teilformeln Ik bis Iz besonders bevorzugt:

$R^1$-Phe-$Z^1$-A-$Z^2$-Phe-$R^2$ Ik

$R^1$-Dio-$Z^1$-A-$Z^2$-Cy-$R^2$ Il

$R^1$-Cy-$Z^1$-A-$Z^2$-Phe-$R^2$ Im

$R^1$-Cy-$Z^1$-A-$Z^2$-Cy-$R^2$ In

$R^1$-Phe-Phe-$Z^1$-A-$Z^2$-$R^2$ Io

$R^1$-Phe-Cy-$Z^1$-A-$Z^2$-$R^2$ Ip

$R^1$-Cy-Phe-$Z^1$-A-$Z^2$-$R^2$ Iq

$R^1$-Cy-Cy-$Z^1$-A-$Z^2$-$R^2$ Ir

$R^1$-Phe-Phe-$Z^1$-A-$Z^2$-Phe-$R^2$ Is

$R^1$-Phe-Phe-$Z^1$-A-$Z^2$-Cy-$R^2$ It

$R^1$-Phe-Cy-$Z^1$-A-$Z^2$-Phe-$R^2$ Iu

$R^1$-Phe-Cy-$Z^1$-A-$Z^2$-Cy-$R^2$ Iv

$R^1$-Cy-Phe-$Z^1$-A-$Z^2$-Phe-$R^2$ Iw

$R^1$-Cy-Phe-$Z^1$-A-$Z^2$-Cy-$R^2$ Ix

$R^1$-Cy-Cy-$Z^1$-A-$Z^2$-Phe-$R^2$ Iy

$R^1$-Cy-Cy-$Z^1$-A-$Z^2$-Cy-$R^2$ Iz

In den Verbindungen der vor- und nachstehenden Formeln bedeuten $R^1$ und $R^2$ vorzugsweise Alkyl, ferner Alkoxy (insbesondere, wenn diese Reste an einer Phe-Gruppe stehen) oder eine andere Oxaalkylgruppe.

$A^1$ und $A^2$ sind bevorzugt Cy oder Phe, ferner bevorzugt Dio oder Pip; bevorzugt enthält die Verbindung der Formel I nicht mehr als einen der Reste Dio, Pip, Bic oder Pyr.

A ist eine 1-X-1,4-cyclohexylengruppe, worin X bevorzugt CN, $CH_3$, $CH_3O$ oder $CF_3$ bedeutet.

$Z^1$ und $Z^2$ sind bevorzugt Einfachbindungen, in zweiter Linie bevorzugt -CO-O- oder -O-CO-Gruppen.

m ist vorzugsweise 1, n ist vorzugsweise 0.

$X^1$ ist vorzugsweise Cl oder Br, aber auch I, OH oder reaktionsfähig verestertes OH wie Alkylsulfonyloxy mit insbesondere 1-6 C-Atomen (z.B. Methylsulfonyloxy) oder Arylsulfonyloxy mit insbesondere 6-10 C-Atomen (z.B. Phenyl-, p-Tolyl- oder Naphthylsulfonyloxy).

Falls $R^1$ und/oder $R^2$ Alkylreste bedeuten, in denen auch eine ("Alkoxy" bzw. "Oxaalkyl") oder zwei ("Alkoxyalkoxy" bzw. "Dioxaalkyl") $CH_2$-Gruppen durch O-Atmone ersetzt sein können, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5- Oxahexyl, 2-, 3-, 4-, 5- oder 6 Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I sowie Ia bis Iap mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Mehtylpentoxy, 2-Ethylhexoxy, 1-Methylhexoxy, 2-Oxa-3-methylbutyl, 3-Oxa-4-methylpentyl.

In den Resten R und X sind die Alkylgruppen bzw. Alkoxygruppen ebenfalls vorzugsweise geradkettig und bedeuten insbesondere Methyl oder Ethyl, ferner Propyl, Butyl oder Pentyl, X auch Methoxy oder Ethoxy, ferner Propoxy, Butoxy oder Pentoxy.

Unter den Verbindungen der Formeln I sowie Ia bis Iz sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Iaa bis Iat:

$R^1$-$A^1$-$Z^1$-A-$R^2$ Iaa

$R^1$-Phe-$Z^1$-A-$R^2$ Iab

$R^1$-Phe-A-$R^2$ Iac

$R^1$-Phe-CO-O-A-$R^2$ Iad

$R^1$-Phe-O-CO-A-$R^2$ Iae

$R^1$-Phe-$CH_2CH_2$-A-$R^2$ Iaf

$R^1$-Phe-O-$CH_2$-A-$R^2$ Iag

$R^1$-Phe-$CH_2$-O-A-$R^2$ Iah

$R^1$-Cy-$Z^1$-A-$R^2$ Iai

$R^1$-Cy-A-$R^2$ Iaj

$R^1$-Cy-CO-O-A-$R^2$ Iak

$R^1$-Cy-O-CO-A-$R^2$ Ial

$R^1$-Cy-$CH_2CH_2$-A-$R^2$ Iam

$R^1$-Cy-O-$CH_2$-A-$R^2$ Ian

$R^1$-Cy-$CH_2$-O-A-$R^2$ Iao

$R^1$-Dio-A-$R^2$ Iap

$R^1$-Pip-A-$R^2$ Iaq

$R^1$-Bic-A-$R^2$ Iar

$R^1$-Pyr-A-$R^2$ Ias

$R^1$-Phe-Phe-A-$R^2$ Iat

Verbindungen der angegebenen Formeln I sowie Ia bis Iat sind besonders bevorzugt, in denen der Rest A

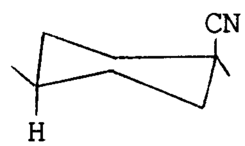

bedeutet.

Diejenigen der vorstehend genannten Formeln, die eine oder mehrere der Gruppen Dio, Pip und/oder Pyr enthalten, umschließen jeweils die beiden möglichen 2,5- bzw. 1,4-Stellungsisomeren. So umschließt beispielsweise die Teilformel Iap die 2-$R^1$-5-(A-$R^2$)-1,3-dioxane und die 2-(A-$R^2$)-5-$R^1$-1,3-dioxane, die Teilformel Iaq die 1-$R^1$-4-(A-$R^2$)-piperidine und die 1-(A-$R^2$)-4-$R^1$-piperidine.

Besonders bevorzugt sind Verbindungen der Formel I, Ia bis Iz sowie Iaa bis Iat, worin $R^1$ und $R^2$ jeweils geradkettige oder höchstens einfach verzweigte Alkylgruppen mit 1-10 C-Atomen, worin auch eine oder zwei $CH_2$-Gruppen durch O-Atome ersetzt sein können, F, Cl, Br, CN oder -O-COR bedeuten und $A^1$, $A^2$, $Z^1$, $Z^2$, m und n die bei Formel I angegebene Bedeutung haben.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber anstelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer -$CH_2CH_2$-Gruppe eine -CH=CH-Gruppe und/oder an Stelle einer -$CH_2$-Gruppe eine -CO-Gruppe und/oder an Stelle eines H-Atoms eine freie oder eine funktionell (z.B. in Form ihres p-Toluolsulfonats) angewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z.B. aus 1-Cyancyclohexenderivaten die entsprechenden Cyclohexanderivate.

Verbindungen der Formel I sind weiterhin erhältlich, indem man an ein entsprechendes Cyclohexenderivat (das der Formel I entspricht, aber anstelle des Restes

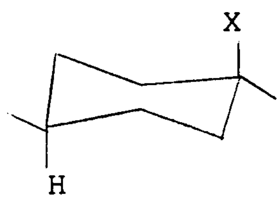

eine 1-Cyclohexen1,4-diylgruppe enthält) eine Verbindung der Formel HX (Fluor-, Chlor-, Brom- oder Cyanwasserstoff) anlagert.

Diese Anlagerung gelingt z.B. in Gegenwart eines inerten Lösungsmittels, z.B. eines halogenierten Kohlenwasserstoffs wie $CH_2Cl_2$ oder $CHCl_3$, eines Nitrils wie Acetonitril oder eines Amids wie Dimethylformamid (DMF) bei Temperaturen zwischen etwa -10 und +150° und Drucken zwischen etwa 1 und 100 bar. Ein Zusatz von Katalysatoren kann günstig sein, z.B. eine HCN-Anlagerung durch Zusatz von Palladium-bis-[2,3-O-isopropyliden-2,3-dihydroxy-1,4-bis-(diphenylphosphino)-butan] katalysiert werden.

Ester der Formel I ($R^1$ und/oder $R^2$ = -O-COR oder $Z^1$ und/oder $Z^2$ = -CO-O- oder -O-CO-) können auch durch Veresterung entsprechender Carbonsäuren der Formeln R-COOH, $R^1-(A^1)_m-COOH$, $R^1-(A^1)_m-Z^1-A-COOH$, $R^2-(A^2)_n-COOH$ oder $R^2-(A^2)_n-Z^2-A-COOH$ (oder ihrer reaktionsfähigen Derivate) mit Alkoholen bzw. Phenolen der Formeln $R^1-(A^1)_m-Z^1-A-Z^2-(A^2)_n-OH$, $R^2-(A^2)_n-Z^2-A-Z^1-(A^1)_m-OH$, $R^2-(A^2)_n-Z^2-A-OH$, $R^2-(A^2)_n-OH$, $R^1-(A^1)_m-Z^1-A-OH$ oder $R^1-(A^1)_m-OH$ (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride der Formeln $R^1-(A^1)_m-CO-O-COCH_3$, $R^1-(A^1)_m-Z^1-A-CO-O-COCH_3$, $R^2-(A^2)_nCO-O-COCH_3$ und $R^1-(A^2)_n-Z^2-A-CO-O-COCH_3$, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate der Formeln $R^1-(A^1)_m-Z^1-A-Z^2-(A^2)_n-OM$, $R^2-(A^2)_n-Z^2-A-Z^1-(A^1)_m-OM$, $R^2(A^2)_n-Z^2-A-OM$, $R^2-(A^2)_n-OM$, $R^1-(A^1)_m-Z^1-A-OM$ und $R^1-(A^1)_m-OM$ in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen - 50° und +250°, vorzugsweise zwischen -20° und +80 °C. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder

Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20 °C.

Dioxanderivate der Formel I (worin eine der Gruppen $A^1$ und/oder $A^2$ eine 1,3-Dioxan-2,5-diyl-Gruppe bedeutet) werden zweckmäßig durch Reaktion eines entsprechenden Aldehyds, z.B. der Formeln $R^1$-$(A^1)_{m-1}$-CHO, $R^1$-$(A^1)_m$-$Z^1$-A-$Z^2$-CHO, O=CH-$(A^1)_{m-1}$-$Z^1$-A-$Z^2$-$(A^2)_n$-$R^2$ bzw. O=CH-$R^2$ (oder eines seiner reaktionsfähigen Derivate) mit einem entsprechenden 1,3-Diol z.B. der Formeln $(HOCH_2)_2CH$-$(A^1)_{m-1}$-$Z^1$-A-$Z^2$-$(A^2)_n$-$R^2$, $(HOCH_2)_2CH$-$R^2$, $R^1$-$(A^1)_{m-1}$-$CH(CH_2OH)_2$ bzw. $R^1$-$(A^1)_m$-$Z^1$-A-$Z^2$-$CH(CH_2OH)_2$ (oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale z.B. der Formeln $R^1$-$(A^1)_{m-1}$-$CH(OR^3)_2$, $R^1$-$(A^1)_m$-$Z^1$-A-$Z^2$-$CH(OR^3)_2$, $(R^3O)_2CH$-$(A^1)_{m-1}$-$Z^1$-A-$Z^2$-$(A^2)_n$-$R^2$, $(R^3O)_2$-$R^2$, $R^4$-$CH(OCH_2)_2CH$-$(A^1)_{m-1}$-$Z^1$-A-$Z^2$-$(A^2)_n$-$R^2$, $R^4$-$CH(OCH_2)_2CH$-$R^2$, $R^1$-$(A^1)_{m-1}$-$CH(CH_2O)_2CH$-$R^4$ bzw. $R^1$-$(A^1)_m$-$Z^1$-A-$Z^1$-$CH(CH_2O)_2CHR^4$, worin $R^3$ Alkyl mit 1-4 C-Atomen, zwei Reste $R^3$ zusammen auch Alkylen mit 2 oder 3 C-Atomen und $R^4$ H, Alkyl mit 1-4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxidation entsprechender Alkohole oder durch Reduktion entsprechender Carbonsäuren oder ihrer Derivate, die Diole durch Reduktion entsprechender Diester erhältlich.

Zur Herstellung von Nitrilen der Formel I (worin $R^1$ und/oder $R^2$ CN bedeuten und/oder X eine CN-Gruppe bedeutet) können entsprechende Säureamide, z.B. solche, in denen an Stelle des Restes X eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ - (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol und Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann' man direkt die Nitrile isolieren.

Nitrile der Formel I, worin X CN bedeutet und worin ferner einer der Reste $Z^1$ bzw. $Z^2$ eine Einfachbindung bedeutet, sind erhältlich, indem man ein Nitril der Formel IV

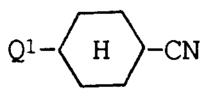

IV

worin

$Q^1$ $R^1$-$(A^1)_m$-$Z^1$-

bedeutet und

$R^1$, $A^1$, $Z^1$ und m die angegebenen Bedeutungen haben,

mit einer Verbindung der Formel V

$Q^2$-$X^1$ V

worin
$Q^2$ $R^2$-$(A^2)_n$-
und $X^1$ Cl, Br, I, OH oder eine reaktionsfähige veresterte OH-Gruppe bedeutet und $R^2$, $A^2$ und n die angegebenen Bedeutungen haben, umsetzt. Die Nitrile der Formel IV sind beispielsweise aus entsprechenden Amiden durch Dehydratisierung erhältlich, die Halogenide der Formel V aus entsprechenden Alkoholen der Formel $Q^2$-OH.

Ether der Formel I (worin $R^1$ und/oder $R^2$ eine Alkylgruppe bedeutet, worin eine oder zwei $CH_2$-Gruppen durch O-Atome ersetzt sind, und/oder worin $Z^1$ und/oder $Z^2$ eine $-OCH_2-$ oder eine $-CH_2O$-Gruppe ist) sind durch Veretherung entsprechender Hydroxyverbindungen, vorzugsweise entsprechender Phenole, erhältlich, wobei die Hydroxyverbindungen zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Verbindungen der Formel I, die $CF_3$-Gruppen enthalten, kann man entsprechende Carbonsäuren, die ihrerseits erhältlich sind, mit $SF_4$ umsetzen, zweckmäßig mit einem Überschuß von $SF_4$ unter Druck in Abwesenheit oder in Gegenwart eines inerten Lösungsmittels wie Cyclohexan oder Dichlormethan bei Temperaturen zwischen etwa 70° und 200°. Die Reaktionszeiten bewegen sich zwischen etwa 2 Stunden und etwa 4 Tagen.

Zur Herstellung von Nitrilen der Formel I (worin $R^1$ und/oder $R^2$ CN bedeuten und/oder worin A durch mindestens eine CN-Gruppe substituiert ist) können auch entsprechende Chlor- oder Bromverbindungen der Formel I (worin $R^1$ und/oder $R^2$ Cl oder Br bedeuten und/oder worin A durch mindestens eine Cl- oder Br-Atom substituiert ist) mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Ebenso ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl-oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel VI charakterisieren,

$R^5$-L-G-E-$R^6$ VI

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH-<br>-CH=CY-<br>-C≡C-<br>-CO-O-<br>-CO-S-<br>-CH=N- | -N(O)=N-<br>-CH=N(O)-<br>$-CH_2-CH_2-$<br>$-CH_2-O-$<br>$-CH_2-S-$<br>-COO-Phe-COO- |

oder eine C-C-Einfachbindung, Y Halogen, vorzugsweise Chlor, oder -CN, und $R^5$ und $R^6$ Alkyl, Alkoxy,

Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^5$ und $R^6$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 100, vorzugsweise 10 bis 100 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erklären, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

Eine Lösung von 30,1 g r-1-Cyan-1-methoxymethyl-cis-4-(p-methoxybenzoylmethyl)-cyclohexan (erhältlich aus Anisol und cis-4-Cyan-4-methoxymethylcyclohexyl-r-1-acetylchlorid in Gegenwart von $AlCl_3$) in 500 ml THF wird an 5 g 10%igem Pd/C bei 40° und 1 bar bis zur Aufnahme von 0,2 Mol $H_2$ hydriert. Man filtriert, dampft ein und erhält r-1-Cyan-1-methoxymethyl-cis-4-(2-p-methoxyphenylethyl)-cyclohexan.

Analog erhält man durch Reduktion der entsprechenden Ketone:
r-1-Methyl-1-propyl-cis-4-(2-p-methoxyphenylethyl)-cyclohexan
r-1-Methyl-1-propyl-cis-4-(2-p-ethoxyphenylethyl)-cyclohexan
r-1-Methyl-1-propyl-cis-4-(2-p-ethylphenylethyl)-cyclohexan
r-1-Methyl-1-propyl-cis-4-(2-p-cyanphenylethyl)-cyclohexan
r-1-Methyl-1-propyl-cis-4-(4'-ethyl-4-biphenylyl)-cyclohexan
(aus der 4'-Acetylverbindung).

**Beispiel 2**

Eine Aufschlämmung von 0,5 g $LiAlH_4$ in 15 ml THF wird bei 10° mit einer Lösung von 4,62 g r-1-p-Toluolsulfonyloxy-methyl-1-butyl-cis-4-(trans-4-butylcyclohexyl)-cyclohexan [F. 95-95,7°; erhältlich durch Hydrolyse von r-1-Cyan-1-butyl-cis-4-(trans-4-butylcyclohexyl)-cyclohexan (s. unten) zur Carbonsäure, Reduktion mit $LiAlH_4$ zu r-1-Hydroxymethyl-1-butyl-cis-4-(trans-4-butylcyclohexyl)-cyclohexan und Tosylierung] in 30 ml THF versetzt und 16 Std. gekocht. Nach üblicher Aufarbeitung erhält man r-1-Methyl-1-butyl-cis-4-(trans-4-butylcyclohexyl)-cyclohexan, F. 0°, K. 85°.

Analog erhält man durch Reduktion der entsprechenden Tosylate:
r-1-Methyl-1-propyl-cis-4-(trans-4-propylcyclohexyl)-cyclohexan
r-1-Methyl-1-propyl-cis-4-(trans-4-butylcyclohexyl)-cyclohexan
r-1-Methyl-1-propyl-cis-4-(trans-4-pentylcyclohexyl)-cyclohexan
r-1-Methyl-1-propyl-cis-4-(trans-4-hexylcyclohexyl)-cyclohexan
r-1-Methyl-1-propyl-cis-4-(trans-4-heptylcyclohexyl)-cyclohexan
r-1-Methyl-1-butyl-cis-4-(trans-4-propylcyclohexyl)-cyclohexan
r-1-Methyl-1-butyl-cis-4-(trans-4-butylcyclohexyl)-cyclohexan
r-1-Methyl-1-butyl-cis-4-(trans-4-pentylcyclohexyl)-cyclohexan
r-1-Methyl-1-butyl-cis-4-(trans-4-hexylcyclohexyl)-cyclohexan

r-1-Methyl-1-butyl-cis-4-(trans-4-heptylcyclohexyl)-cyclohexan
r-1-Methyl-1-pentyl-cis-4-(trans-4-propylcyclohexyl)-cyclohexan
r-1-Methyl-1-pentyl-cis-4-(trans-4-butylcyclohexyl)-cyclohexan
r-1-Methyl-1-pentyl-cis-4-(trans-4-pentylcyclohexyl)-cyclohexan
r-1-Methyl-1-pentyl-cis-4-(trans-4-hexylcyclohexyl)-cyclohexan
r-1-Methyl-1-pentyl-cis-4-(trans-4-heptylcyclohexyl)-cyclohexan
r-1-Methyl-1-hexyl-cis-4-(trans-4-propylcyclohexyl)-cyclohexan
r-1-Methyl-1-hexyl-cis-4-(trans-4-butylcyclohexyl)-cyclohexan
r-1-Methyl-1-hexyl-cis-4-(trans-4-pentylcyclohexyl)-cyclohexan
r-1-Methyl-1-hexyl-cis-4-(trans-4-hexylcyclohexyl)-cyclohexan
r-1-Methyl-1-hexyl-cis-4-(trans-4-heptylcyclohexyl)-cyclohexan
r-1-Methyl-1-heptyl-cis-4-(trans-4-propylcyclohexyl)-cyclohexan
r-1-Methyl-1-heptyl-cis-4-(trans-4-butylcyclohexyl)-cyclohexan
r-1-Methyl-1-heptyl-cis-4-(trans-4-pentylcyclohexyl)-cyclohexan
r-1-Methyl-1-heptyl-cis-4-(trans-4-hexylcyclohexyl)-cyclohexan
r-1-Methyl-1-heptyl-cis-4-(trans-4-heptylcyclohexyl)-cyclohexan

**Beispiel 3**

Ein Gemisch von 27,4 g r-1-Acetyl-1-propyl-cis-4-p-methoxyphenylcyclohexan (erhältlich aus r-1-Cyan-1-propyl-cis-4-p-methoxyphenylcyclohexan und $CH_3MgJ$), 15 g KOH, 30 ml 85%igem Hydrazin und 250 ml Triethylenglykol wird 1 Std. auf 120° erhitzt. Man steigert die Temperatur bis zur Zersetzung des gebildeten Hydratzons, kocht noch 4 Std., kühlt ab, arbeitet wir üblich auf und erhält r-1-Ethyl-1-propyl-cis-4-p-methoxyphenylcyclohexan.

Analog erhält man durch Wolff-Kishner-Reduktion der entsprechenden Ketone:
r-1-Methyl-1-propyl-cis-4-(trans-4-p-ethylphenylcyclohexyl)-cyclohexan
(aus der p-Acetylphenyl-Verbindung)
r-1-Ethyl-1-propyl-cis-4-(trans-4-propylcyclohexyl)-cyclohexan
r-1-Ethyl-1-propyl-cis-4-(trans-4-butylcyclohexyl)-cyclohexan
r-1-Ethyl-1-propyl-cis-4-(trans-4-pentylcyclohexyl)-cyclohexan
r-1-Ethyl-1-propyl-cis-4-(trans-4-hexylcyclohexyl)-cyclohexan
r-1-Ethyl-1-propyl-cis-4-(trans-4-heptylcyclohexyl)-cyclohexan
r-1-Ethyl-1-butyl-cis-4-(trans-4-propylcyclohexyl)-cyclohexan
r-1-Ethyl-1-butyl-cis-4-(trans-4-butylcyclohexyl)-cyclohexan
r-1-Ethyl-1-butyl-cis-4-(trans-4-pentylcyclohexyl)-cyclohexan
r-1-Ethyl-1-butyl-cis-4-(trans-4-hexylcyclohexyl)-cyclohexan
r-1-Ethyl-1-butyl-cis-4-(trans-4-heptylcyclohexyl)-cyclohexan
r-1-Ethyl-1-pentyl-cis-4-(trans-4-propylcyclohexyl)-cyclohexan
r-1-Ethyl-1-pentyl-cis-4-(trans-4-butylcyclohexyl)-cyclohexan
r-1-Ethyl-1-pentyl-cis-4-(trans-4-pentylcyclohexyl)-cyclohexan
r-1-Ethyl-1-pentyl-cis-4-(trans-4-hexylcyclohexyl)-cyclohexan
r-1-Ethyl-1-pentyl-cis-4-(trans-4-heptylcyclohexyl)-cyclohexan
r-1-Ethyl-1-hexyl-cis-4-(trans-4-propylcyclohexyl)-cyclohexan
r-1-Ethyl-1-hexyl-cis-4-(trans-4-butylcyclohexyl)-cyclohexan
r-1-Ethyl-1-hexyl-cis-4-(trans-4-pentylcyclohexyl)-cyclohexan
r-1-Ethyl-1-hexyl-cis-4-(trans-4-hexylcyclohexyl)-cyclohexan
r-1-Ethyl-1-hexyl-cis-4-(trans-4-heptylcyclohexyl)-cyclohexan
r-1-Ethyl-1-heptyl-cis-4-(trans-4-propylcyclohexyl)-cyclohexan
r-1-Ethyl-1-heptyl-cis-4-(trans-4-butylcyclohexyl)-cyclohexan
r-1-Ethyl-1-heptyl-cis-4-(trans-4-pentylcyclohexyl)-cyclohexan
r-1-Ethyl-1-heptyl-cis-4-(trans-4-hexylcyclohexyl)-cyclohexan
r-1-Ethyl-heptyl-cis-4-(trans-4-heptylcyclohexyl)-cyclohexan
1,1-Dipropyl-4-(trans-4-propylcyclohexyl)-cyclohexan.

**Beispiel 4**

Eine Lösung von 30,4 g r-1-Fluor-1-pentyl-cis-4-(2-p-methoxyphenylvinyl)-cyclohexan [erhältlich durch Reaktion von 4-Fluor-4-pentylcyclohexylmagnesiumbromid mit p-Methoxyphenylacetaldehyd, nachfolgende

Hydrolyse zu r-1-Fluor-1-pentyl-cis-4-(2-p-methoxyphenyl-1-hydroxyethyl)-cyclohexan und Dehydratisierung mit p-Toluolsulfonsäure in siedendem Toluol] in 600 ml THF wird an 5 g PdO bei 40° und 1 bar bis zur Aufnahme von 0,1 Mol $H_2$ hydriert. Man filtriert, dampft ein und erhält r-1-Fluor-1-pentyl-cis-4-(2-p-methoxyphenylethyl)-cyclohexan.

Analog erhält man durch Hydrierung der entsprechenden Ethylenderivate bzw. Cyclohexenderivate:
r-1-Chlor-1-pentyl-cis-4-(2-p-methoxyphenylethyl)-cyclohexan
r-1-Brom-1-pentyl-cis-4-(2-p-methoxyphenylethyl)-cyclohexan
r-1-Methyl-1-propyl-cis-4-p-methoxyphenyl-cyclohexan (aus 1-p-Methoxyphenyl-4-methyl-4-propyl-cyclohexen)
r-1-Methyl-1-butyl-cis-4-p-methoxyphenyl-cyclohexan
r-1-Methyl-1-pentyl-cis-4-p-methoxyphenyl-cyclohexan
r-1-Methyl-1-hexyl-cis-4-p-methoxyphenyl-cyclohexan
r-1-Methyl-1-heptyl-cis-4-p-methoxyphenyl-cyclohexan
r-1-Methyl-1-butyl-cis-4-p-ethoxyphenyl-cyclohexan
r-1-Methyl-1-pentyl-cis-4-p-ethoxyphenyl-cyclohexan
r-1-Methyl-1-hexyl-cis-4-p-ethoxyphenyl-cyclohexan
r-1-Methyl-1-heptyl-cis-4-p-ethoxyphenyl-cyclohexan
r-1-Methyl-1-butyl-cis-4-p-ethylphenyl-cyclohexan
r-1-Methyl-1-pentyl-cis-4-p-ethylphenyl-cyclohexan
r-1-Methyl-1-hexyl-cis-4-p-ethylphenyl-cyclohexan
r-1-Methyl-1-heptyl-cis-4-p-ethylphenyl-cyclohexan
r-1-Methyl-1-butyl-cis-4-p-cyanphenyl-cyclohexan
r-1-Methyl-1-pentyl-cis-4-p-cyanphenyl-cyclohexan
r-1-Methyl-1-hexyl-cis-4-p-cyanphenyl-cyclohexan
r-1-Methyl-1-heptyl-cis-4-p-cyanphenyl-cyclohexan
r-1-Methyl-1-propyl-cis-4-[trans-4-(trans-4-propylcyclohexyl)-cyclohexyl]-cyclohexan.

**Beispiel 5**

Man leitet bei 0° HBr in eine Lösung von 2,42 g 1-Propyl-4-p-propylphenylcyclohexen (erhältlich durch Reaktion von 4-p-Propylphenylcyclohexanon mit $C_3H_7MgBr$, Hydrolyse zu 1-Propyl-4-p-propylphenylcyclohexan-1-ol und Dehydratisierung) in 50 ml $CH_2Cl_2$, läßt über Nacht stehen, arbeitet wie üblich auf und erhält r-1-Brom-1-propyl-cis-4-p-propylphenylcyclohexan.

Analog erhält man durch Anlagerung von HCl bzw. HBr an die entsprechenden Cyclohexene:
r-1-Chlor-1-pentyl-cis-4-p-methoxyphenylcyclohexan
r-1-Brom-1-pentyl-cis-4-p-methoxyphenylcyclohexan

**Beispiel 6**

In einem Autoklaven erhitzt man 30,4 g 1-Pentyl-4-(trans-4-pentylcyclohexyl)-cyclohexen [erhältlich durch Reaktion von 4-(trans-4-Pentylcyclohexyl)-cyclohexanon mit Pentyl-MgBr, Hydrolyse zu 1-Pentyl-4-(trans-4-pentylcyclohexyl)-cyclohexanol und Dehydratisierung], 2,7 g flüssiges HCN, 0,1 g Palladium-bis-[2,3-O-isopropyliden-2,3-dihydroxy-1,4-bis-(diphenylphosphino)-butan] und 100 ml Acetonitril 1 Std. auf 130°. Nach dem Abkühlen, Eindampfen und üblicher Aufarbeitung erhält man r-1-Cyan-1-pentyl-cis-4-(trans-4-pentyl-cyclohexyl)-cyclohexan, F. 28°, K. 63°.

Analog erhält man durch Anlagerung von HCN an die entsprechenden Cyclohexenderivate:
r-1-Cyan-1-propyl-cis-4-(4-propyl-1-piperidyl)-cyclohexan
r-1-Cyan-1-propyl-cis-4-(4-butyl-1-piperidyl)-cyclohexan
r-1-Cyan-1-propyl-cis-4-(4-pentyl-1-piperidyl)-cyclohexan
r-1-Cyan-1-propyl-cis-4-(4-hexyl-1-piperidyl)-cyclohexan
r-1-Cyan-1-propyl-cis-4-(4-heptyl-1-piperidyl)-cyclohexan
r-1-Cyan-1-butyl-cis-4-(4-propyl-1-piperidyl)-cyclohexan
r-1-Cyan-1-butyl-cis-4-(4-butyl-1-piperidyl)-cyclohexan
r-1-Cyan-1-butyl-cis-4-(4-pentyl-1-piperidyl)-cyclohexan
r-1-Cyan-1-butyl-cis-4-(4-hexyl-1-piperidyl)-cyclohexan
r-1-Cyan-1-butyl-cis-4-(4-heptyl-1-piperidyl)-cyclohexan
r-1-Cyan-1-pentyl-cis-4-(4-propyl-1-piperidyl)-cyclohexan
r-1-Cyan-1-pentyl-cis-4-(4-butyl-1-piperidyl)-cyclohexan

r-1-Cyan-1-pentyl-cis-4-(4-pentyl-1-piperidyl)-cyclohexan
r-1-Cyan-1-pentyl-cis-4-(4-hexyl-1-piperidyl)-cyclohexan
r-1-Cyan-1-pentyl-cis-4-(4-heptyl-1-piperidyl)-cyclohexan
r-1-Cyan-1-hexyl-cis-4-(4-propyl-1-piperidyl)-cyclohexan
r-1-Cyan-1-hexyl-cis-4-(4-butyl-1-piperidyl)-cyclohexan
r-1-Cyan-1-hexyl-cis-4-(4-pentyl-1-piperidyl)-cyclohexan
r-1-Cyan-1-hexyl-cis-4-(4-hexyl-1-piperidyl)-cyclohexan
r-1-Cyan-1-hexyl-cis-4-(4-heptyl-1-piperidyl)-cyclohexan
r-1-Cyan-1-heptyl-cis-4-(4-propyl-1-piperidyl)-cyclohexan
r-1-Cyan-1-heptyl-cis-4-(4-butyl-1-piperidyl)-cyclohexan
r-1-Cyan-1-heptyl-cis-4-(4-pentyl-1-piperidyl)-cyclohexan
r-1-Cyan-1-heptyl-cis-4-(4-hexyl-1-piperidyl)-cyclohexan
r-1-Cyan-1-heptyl-cis-4-(4-heptyl-1-piperidyl)-cyclohexan
r-1-Cyan-1-propyl-cis-4-(4-propyl-bicyclo[2,2,2]octyl)-cyclohexan
r-1-Cyan-1-propyl-cis-4-(4-butyl-bicyclo[2,2,2]octyl)-cyclohexan
r-1-Cyan-1-propyl-cis-4-(4-pentyl-bicyclo[2,2,2)octyl)-cyclohexan
r-1-Cyan-1-propyl-cis-4-(4-hexyl-bicyclo[2,2,2]octyl)-cyclohexan
r-1-Cyan-1-propyl-cis-4-(4-heptyl-bicyclo[2,2,2]octyl)-cyclohexan
r-1-Cyan-1-butyl-cis-4-(4-propyl-bicyclo[2,2,2]octyl)-cyclohexan
r-1-Cyan-1-butyl-cis-4-(4-butyl-bicyclo[2,2,2]octyl)-cyclohexan
r-1-Cyan-1-butyl-cis-4-(4-pentyl-bicyclo[2,2,2]octyl)-cyclohexan
r-1-Cyan-1-butyl-cis-4-(4-hexyl-bicyclo[2,2,2]octyl)-cyclohexan
r-1-Cyan-1-butyl-cis-4-(4-heptyl-bicyclo[2,2,2]octyl)-cyclohexan
r-1-Cyan-1-pentyl-cis-4-(4-propyl-bicyclo[2,2,2]octyl)-cyclohexan
r-1-Cyan-1-pentyl-cis-4-(4-butyl-bicyclo[2,2,2]octyl)-cyclohexan
r-1-Cyan-1-pentyl-cis-4-(4-pentyl-bicyclo[2,2,2]octyl)-cyclohexan
r-1-Cyan-1-pentyl-cis-4-(4-hexyl-bicyclo[2,2,2]octyl)-cyclohexan
r-1-Cyan-1-pentyl-cis-4-(4-heptyl-bicyclo[2,2,2]octyl)-cyclohexan
r-1-Cyan-1-hexyl-cis-4-(4-propyl-bicyclo[2,2,2]octyl)-cyclohexan
r-1-Cyan-1-hexyl-cis-4-(4-butyl-bicyclo[2,2,2]octyl)-cyclohexan
r-1-Cyan-1-hexyl-cis-4-(4-pentyl-bicyclo[2,2,2]octyl)-cyclohexan
r-1-Cyan-1-hexyl-cis-4-(4-hexyl-bicyclo[2,2,2]octyl)-cyclohexan
r-1-Cyan-1-hexyl-cis-4-(4-heptyl-bicyclo[2,2,2]octyl)-cyclohexan
r-1-Cyan-1-heptyl-cis-4-(4-propyl-bicyclo[2,2,2]octyl)-cyclohexan
r-1-Cyan-1-heptyl-cis-4-(4-butyl-bicyclo[2,2,2]octyl)-cyclohexan
r-1-Cyan-1-heptyl-cis-4-(4-pentyl-bicyclo[2,2,2]octyl)-cyclohexan
r-1-Cyan-1-heptyl-cis-4-(4-hexyl-bicyclo[2,2,2]octyl)-cyclohexan
r-1-Cyan-1-heptyl-cis-4-(4-heptyl-bicyclo[2,2,2]octyl)-cyclohexan

**Beispiel 7**

Man kocht 17 g trans-4-Propylcyclohexancarbonsäure 1 Std. mit 24 g $SOCl_2$, dampft ein, löst das erhaltene rohe Säurechlorid in 150 ml Toluol, versetzt mit 8 ml Pyridin und 16,7 g cis-4-Cyan-4-propylcyclohexanol (erhältlich durch Alkylierung von 4-Cyancyclohexanol) und kocht 2 Stunden. Nach Abkühlen und üblicher Aufarbeitung erhält man trans-4-Propylcyclohexancarbonsäure-(cis-4-cyan-4-propylcyclohexylester).

Analog erhält man durch Veresterung der entsprechenden Säuren:
trans-4-Propylcyclohexancarbonsäure-(cis-4-cyan-4-butylcyclohexylester)
trans-4-Propylcyclohexancarbonsäure-(cis-4-cyan-4-pentylcyclohexylester)
trans-4-Propylcyclohexancarbonsäure-(cis-4-cyan-4-hexylcyclohexylester)
trans-4-Propylcyclohexancarbonsäure-(cis-4-cyan-4-heptylcyclohexylester)
trans-4-Butylcyclohexancarbonsäure-(cis-4-cyan-4-propylcyclohexylester)
trans-4-Butylcyclohexancarbonsäure-(cis-4-cyan-4-butylcyclohexylester)
trans-4-Butylcyclohexancarbonsäure-(cis-4-cyan-4-pentylcyclohexylester)
trans-4-Butylcyclohexancarbonsäure-(cis-4-cyan-4-hexylcyclohexylester)
trans-4-Butylcyclohexancarbonsäure-(cis-4-cyan-4-heptylcyclohexylester)
trans-4-Pentylcyclohexancarbonsäure-(cis-4-cyan-4-propylcyclohexylester)
trans-4-Pentylcyclohexancarbonsäure-(cis-4-cyan-4-butylcyclohexylester)

trans-4-Pentylcyclohexancarbonsäure-(cis-4-cyan-4-pentylcyclohexylester)
trans-4-Pentylcyclohexancarbonsäure-(cis-4-cyan-4-hexylcyclohexylester)
trans-4-Pentylcyclohexancarbonsäure-(cis-4-cyan-4-heptylcyclohexylester)
trans-4-Hexylcyclohexancarbonsäure-(cis-4-cyan-4-propylcyclohexylester)
trans-4-Hexylcyclohexancarbonsäure-(cis-4-cyan-4-butylcyclohexylester)
trans-4-Hexylcyclohexancarbonsäure(cis-4-cyan-4-pentylcyclohexylester)
trans-4-Hexylcyclohexancarbonsäure-(cis-4-cyan-4-hexylcyclohexylester)
trans-4-Hexylcyclohexancarbonsäure-(cis-4-cyan-4-heptylcyclohexylester)
trans-4-Heptylcyclohexancarbonsäure-(cis-4-cyan-4-propylcyclohexylester)
trans-4-Heptylcyclohexancarbonsäure-(cis-4-cyan-4-butylcyclohexylester)
trans-4-Heptylcyclohexancarbonsäure-(cis-4-cyan-4-pentylcyclohexylester)
trans-4-Heptylcyclohexancarbonsäure-(cis-4-cyan-4-hexylcyclohexylester)
trans-4-Heptylcyclohexancarbonsäure-(cis-4-cyan-4-heptylcyclohexylester)
cis-4-Cyan-4-propylcyclohexancarbonsäure-(trans-4-propylcyclohexylester)
cis-4-Cyan-4-propylcyclohexancarbonsäure-(trans-4-butylcyclohexylester)
cis-4-Cyan-4-propylcyclohexancarbonsäure-(trans-4-pentylcyclohexylester)
cis-4-Cyan-4-propylcyclohexancarbonsäure-(trans-4-hexylcyclohexylester)
cis-4-Cyan-4-propylcyclohexancarbonsäure-(trans-4-heptylcyclohexylester)
cis-4-Cyan-4-butylcyclohexancarbonsäure-(trans-4-propylcyclohexylester)
cis-4-Cyan-4-butylcyclohexancarbonsäure-(trans-4-butylcyclohexylester)
cis-4-Cyan-4-butylcyclohexancarbonsäure-(trans-4-pentylcyclohexylester)
cis-4-Cyan-4-butylcyclohexancarbonsäure-(trans-4-hexylcyclohexylester)
cis-4-Cyan-4-butylcyclohexancarbonsäure-(trans-4-heptylcyclohexylester)
cis-4-Cyan-4-pentylcyclohexancarbonsäure-(trans-4-propylcyclohexylester)
cis-4-Cyan-4-pentylcyclohexancarbonsäure-(trans-4-butylcyclohexylester)
cis-4-Cyan-4-pentylcyclohexancarbonsäure-(trans-4-pentylcyclohexylester)
cis-4-Cyan-4-pentylcyclohexancarbonsäure-(trans-4-hexylcyclohexylester)
cis-4-Cyan-4-pentylcyclohexancarbonsäure-(trans-4-heptylcyclohexylester)
cis-4-Cyan-4-hexylcyclohexancarbonsäure-(trans-4-propylcyclohexylester)
cis-4-Cyan-4-hexylcyclohexancarbonsäure-(trans-4-butylcyclohexylester)
cis-4-Cyan-4-hexylcyclohexancarbonsäure-(trans-4-pentylcyclohexylester)
cis-4-Cyan-4-hexylcyclohexancarbonsäure-(trans-4-hexylcyclohexylester)
cis-4-Cyan-4-hexylcyclohexancarbonsäure-(trans-4-heptylcyclohexylester)
cis-4-Cyan-4-heptylcyclohexancarbonsäure-(trans-4-propylcyclohexylester)
cis-4-Cyan-4-heptylcyclohexancarbonsäure-(trans-4-butylcyclohexylester)
cis-4-Cyan-4-heptylcyclohexancarbonsäure-(trans-4-pentylcyclohexylester)
cis-4-Cyan-4-heptylcyclohexancarbonsäure-(trans-4-hexylcyclohexylester)
cis-4-Cyan-4-heptylcyclohexancarbonsäure-(trans-4-heptylcyclohexylester)
p-Methoxybenzoesäure-(cis-4-cyan-4-propylcyclohexylester)
p-Methoxybenzoesäure-(cis-4-cyan-4-butylcyclohexylester)
p-Methoxybenzoesäure-(cis-4-cyan-4-pentylcyclohexylester)
p-Methoxybenzoesäure-(cis-4-cyan-4-hexylcyclohexylester)
p-Methoxybenzoesäure-(cis-4-cyan-4-heptylcyclohexylester)
p-Ethoxybenzoesäure-(cis-4-cyan-4-propylcyclohexylester)
p-Ethoxybenzoesäure-(cis-4-cyan-4-butylcyclohexylester)
p-Ethoxybenzoesäure-(cis-4-cyan-4-pentylcyclohexylester)
p-Ethoxybenzoesäure-(cis-4-cyan-4-hexylcyclohexylester)
p-Ethoxybenzoesäure-(cis-4-cyan-4-heptylcyclohexylester)
p-Propoxybenzoesäure-(cis-4-cyan-4-propylcyclohexylester)
p-Propoxybenzoesäure-(cis-4-cyan-4-butylcyclohexylester)
p-Propoxybenzoesäure-(cis-4-cyan-4-pentylcyclohexylester)
p-Propoxybenzoesäure-(cis-4-cyan-4-hexylcyclohexylester)
p-Propoxybenzoesäure-(cis-4-cyan-4-heptylcyclohexylester)
p-Butoxybenzoesäure-(cis-4-cyan-4-propylcyclohexylester)
p-Butoxybenzoesäure-(cis-4-cyan-4-butylcyclohexylester)
p-Butoxybenzoesäure-(cis-4-cyan-4-pentylcyclohexylester)
p-Butoxybenzoesäure-(cis-4-cyan-4-hexylcyclohexylester)
p-Butoxybenzoesäure-(cis-4-cyan-4-heptylcyclohexylester)

p-Propylbenzoesäure-(cis-4-cyan-4-propylcyclohexylester)
p-Propylbenzoesäure-(cis-4-cyan-4-butylcyclohexylester)
p-Propylbenzoesäure-(cis-4-cyan-4-pentylcyclohexylester)
p-Propylbenzoesäure-(cis-4-cyan-4-hexylcyclohexylester)
p-Propylbenzoesäure-(cis-4-cyan-4-heptylcyclohexylester)
p-Butylbenzoesäure-(cis-4-cyan-4-propylcyclohexylester)
p-Butylbenzoesäure-(cis-4-cyan-4-butylcyclohexylester)
p-Butylbenzoesäure-(cis-4-cyan-4-pentylcyclohexylester)
p-Butylbenzoesäure-(cis-4-cyan-4-hexylcyclohexylester)
p-Butylbenzoesäure-(cis-4-cyan-4-heptylcyclohexylester)
p-Pentylbenzoesäure-(cis-4-cyan-4-propylcyclohexylester)
p-Pentylbenzoesäure-(cis-4-cyan-4-butylcyclohexylester)
p-Pentylbenzoesäure-(cis-4-cyan-4-pentylcyclohexylester)
p-Pentylbenzoesäure-(cis-4-cyan-4-hexylcyclohexylester)
p-Pentylbenzoesäure-(cis-4-cyan-4-heptylcyclohexylester)
cis-4-Cyan-4-propylcyclohexancarbonsäure-(p-methoxyphenylester)
cis-4-Cyan-4-propylcyclohexancarbonsäure-(p-ethoxyphenylester)
cis-4-Cyan-4-propylcyclohexancarbonsäur-(p-propoxyphenylester)
cis-4-Cyan-4-propylcyclohexancarbonsäur-(p-butoxyphenylester)
cis-4-Cyan-4-propylcyclohexancarbonsäur-(p-propylphenylester)
cis-4-Cyan-4-propylcyclohexancarbonsäure-(p-butylphenylester)
cis-4-Cyan-4-propylcyclohexancarbonsäur-(p-pentylphenylester)
cis-4-Cyan-4-butylcyclohexancarbonsäure-(p-methoxyphenylester)
cis-4-Cyan-4-butylcyclohexancarbonsäure-(p-ethoxyphenylester)
cis-4-Cyan-4-butylcyclohexancarbonsäure-(p-propoxyphenylester)
cis-4-Cyan-4-butylcyclohexancarbonsäure-(p-butoxyphenylester)
cis-4-Cyan-4-butylcyclohexancarbonsäure-(p-propylphenylester)
cis-4-Cyan-4-butylcyclohexancarbonsäure-(p-butylphenylester)
cis-4-Cyan-4-butylcyclohexancarbonsäure-(p-pentylphenylester)
cis-4-Cyan-4-pentylcyclohexancarbonsäure-(p-methoxyphenylester)
cis-4-Cyan-4-pentylcyclohexancarbonsäure-(p-ethoxyphenylester)
cis-4-Cyan-4-pentylcyclohexancarbonsäure-(p-propoxyphenylester)
cis-4-Cyan-4-pentylcyclohexancarbonsäure-(p-butoxyphenylester)
cis-4-Cyan-4-pentylcyclohexancarbonsäure-(p-propylphenylester)
cis-4-Cyan-4-pentylcyclohexancarbonsäure-(p-butylphenylester)
cis-4-Cyan-4-pentylcyclohexancarbonsäure-(p-pentylphenylester)
cis-4-Cyan-4-hexylcyclohexancarbonsäure-(p-methoxyphenylester)
cis-4-Cyan-4-hexylcyclohexancarbonsäure-(p-ethoxyphenylester)
cis-4-Cyan-4-hexylcyclohexancarbonsäure-(p-propoxyphenylester)
cis-4-Cyan-4-hexylcyclohexancarbonsäure-(p-butoxyphenylester)
cis-4-Cyan-4-hexlcyclohexancarbonsäure-(p-propylphenylester)
cis-4-Cyan-4-hexylcyclohexancarbonsäure-(p-butylphenylester)
cis-4-Cyan-4-hexylcyclohexancarbonsäure-(p-pentylphenylester)
cis-4-Cyan-4-heptylcyclohexancarbonsäure-(p-methoxyphenylester)
cis-4-Cyan-4-heptylcyclohexancarbonsäure-(p-ethoxyphenylester)
cis-4-Cyan-4-heptylcyclohexancarbonsäure-(p-propoxyphenylester)
cis-4-Cyan-4-heptylcyclohexancarbonsäure-(p-butoxyphenylester)
cis-4-Cyan-4-heptylcyclohexancarbonsäure-(p-propylphenylester)
cls-4-Cyan-4-heptylcyclohexancarbonsäure-(p-butylphenylester)
cis-4-Cyan-4-heptylcyclohexancarbonsäure-(p-pentylphenylester)
r-1-Methyl-1-propyl-cyclohexan-cis-4-carbonsäure-(p-methoxyphenylester)
r-1-Methyl-1-propyl-cyclohexan-cis-4-carbonsäure-(p-ethoxyphenylester)
r-1-Methyl-1-propyl-cyclohexan-cis-4-carbonsäure-(trans-4-propylcyclohexylester)
r-1-Methyl-1-propyl-cyclohexan-cis-4-carbonsäure-(trans-4-pentylcyclohexylester)
r-1-Cyan-1-propyl-cis-4-(trans-4-acetoxycyclohexyl)-cyclohexan
r-1-Cyan-1-propyl-cis-4-(trans-4-propionyloxycyclohexyl)-cyclohexan
r-1-Cyan-1-propyl-cis-4-(trans-4-butyryloxycyclohexyl)-cyclohexan
r-1-Cyan-1-propyl-cis-4-(trans-4-valeryloxycyclohexyl)-cyclohexan

r-1-Cyan-1-propyl-cis-4-(trans-4-capronylloxycyclohexyl)-cyclohexan

**Beispiel 8**

Ein Gemisch von 1,2 g 2-Propylpropan-1,3-diol, 1,79 g 1-Cyan-1-propyl-4-formyl-cyclohexan (erhältlich durch Alkylierung von 1-Cyan-4-formylcyclohexan mit Propylbromid), 0,01 g p-Toluolsulfonsäure und 15 ml Toluol wird am Wasserabscheider 3 Std. gekocht, abkühlt, mit Wasser gewaschen und eingedampft. Man erhält 1-r-Cyan-1-propyl-cis-4-(trans-5-propyl-1,3-dioxan-2-yl)-cyclohexan.

Analog erhält man durch Umsetzung der entsprechenden Aldehyde mit den entsprechenden Diolen:
1-r-Cyan-1-propyl-cis-4-(trans-5-butyl-1,3-dioxan-2-yl)-cyclohexan
1-r-Cyan-1-propyl-cis-4-(trans-5-pentyl-1,3-dioxan-2-yl)-cyclohexan
1-r-Cyan-1-propyl-cis-4-(trans-5-hexyl-1,3-dioxan-2-yl)-cyclohexan
1-r-Cyan-1-propyl-cis-4-(trans-5-heptyl-1,3-dioxan-2-yl)-cyclohexan
1-r-Cyan-1-butyl-cis-4-(trans-5-propyl-1,3-dioxan-2-yl)-cyclohexan
1-r-Cyan-1-butyl-cis-4-(trans-5-butyl-1,3-dioxan-2-yl)-cyclohexan
1-r-Cyan-1-butyl-cis-4-(trans-5-pentyl-1,3-dioxan-2-yl)-cyclohexan
1-r-Cyan-1-butyl-cis-4-(trans-5-hexyl-1,3-dioxan-2-yl)-cyclohexan
1-r-Cyan-1-butyl-cis-4-(trans-5-heptyl-1,3-dioxan-2-yl)-cyclohexan
1-r-Cyan-1-pentyl-cis-4-(trans-5-propyl-1,3-dioxan-2-yl)-cyclohexan
1-r-Cyan-1-pentyl-cis-4-(trans-5-butyl-1,3-dioxan-2-yl)-cyclohexan
1-r-Cyan-1-pentyl-cis-4-(trans-5-pentyl-1,3-dioxan-2-yl)-cyclohexan
1-r-Cyan-1-pentyl-cis-4-(trans-5-hexyl-1,3-dioxan-2-yl)-cyclohexan
1-r-Cyan-1-pentyl-cis-4-(trans-5-heptyl-1,3-dioxan-2-yl)-cyclohexan
1-r-Cyan-1-hexyl-cis-4-(tran--5-propyl-1,3-dioxan-2-yl)-cyclohexan
1-r-Cyan-1-hexyl-cis-4-(trans-5-butyl-1,3-dioxan-2-yl)-cyclohexan
1-r-Cyan-1-hexyl-cis-4-(trans-5-pentyl-1,3-dioxan-2-yl)-cyclohexan
1-r-Cyan-1-hexyl-cis-4-(trans-5-hexyl-1,3-dioxan-2-yl)-cyclohexan
1-r-Cyan-1-hexyl-cis-4-(trans-5-heptyl-1,3-dioxan-2-yl)-cyclohexan
1-r-Cyan-1-heptyl-cis-4-(trans-5-propyl-1,3-dioxan-2-yl)-cyclohexan
1-r-Cyan-1-heptyl-cis-4-(trans-5-butyl-1,3-dioxan-2-yl)-cyclohexan
1-r-Cyan-1-heptyl-cis-4-(trans-5-pentyl-1,3-dioxan-2-yl)-cyclohexan
1-r-Cyan-1-heptyl-cis-4-(trans-5-hexyl-1,3-dioxan-2-yl)-cyclohexan
1-r-Cyan-1-heptyl-cis-4-(trans-5-heptyl-1,3-dioxan-2-yl)-cyclohexan.

**Beispiel 9**

Eine Lösung von 34,9 g r-1-Carbamoyl-1-pentyl-cis-4-(trans-4-pentylcyclohexyl)-cyclohexan (erhältlich aus dem Säurechlorid mit $NH_3$) in 500 ml DMF wird bei 50° unter Rühren tropfenweise mit 65 g $POCl_3$ versetzt. Nach weiterem einstündigem Rühren gießt man auf Eis, arbeitet wie üblich auf und erhält r-1-Cyan-1-pentyl-cis-4-(trans-4-pentylcyclohexyl)-cyclohexan, F. 28°, K. 63°.

Analog erhält man durch Wasserabspaltung aus den entsprechenden Amiden:
1-r-Methyl-1-propyl-cis-4-(4'-cyan-4-biphenylyl)-cyclohexan
1-r-Methyl-1-propyl-cis-4-(trans-4-p-cyanphenyl-cyclohexyl)-cyclohexan
1-r-Methyl-1-propyl-cis-4-[trans-4-(trans-4-cyancyclohexyl)-cyclohexyl]-cyclohexan.

**Beispiel 10**

Eine Lösung von 36,9 g 1-Pentyl-cis-4-(trans-4-pentylcyclohexyl)-cyclohexan-r-1-carbonylchlorid [erhältlich durch Reaktion von 4-(4-Pentylcyclohexyl)-cyclohexanon mit Pentyl-Li und nachfolgende Hydrolyse zu 1-Pentyl-4-(4-pentylcyclohexyl)-cyclohexanol,Umsetzung mit K, dann mit $CO_2$ zu 1-Pentyl-cis-4-(trans-4-pentylcyclohexyl)-cyclohexan-r-1-carbonsäure und Reaktion mit $SOCl_2$] und 8 g Sulfamid in 500 ml Tetramethylensulfon wird 4 Std. auf 120° erhitzt, eingedampft und wie üblich aufgearbeitet. Man erhält r-1-Cyan-1-pentyl-cis-4-(trans-4-pentylcyclohexyl)-cyclohexan, F. 28°, K. 63°.

**Referenzbeispiel 11**

Eine Lösung von 26,3 g 4'-Pentyl-biphenylyl-4-acetonitril [F. 80°, K. -10°; erhältlich aus 4-Acetyl-4'-pentyl-biphenyl über 4'-Pentyl-biphenylyl-4-essigsäure (F. 160°) und das entsprechende Amid (F. 185°)] in

150 ml Dimethylsulfoxid wird unter Rühren mit 6 g NaH (50%ig in Paraffin), dann mit einer Lösung von 25,3 g 1,5-Dibrompentan in 50 ml Dioxan versetzt; dabei hält man die Temperatur durch Kühlen unter 35°. Man rührt noch 2 Std., versetzt mit Isopropanol und arbeitet wie üblich auf. Man erhält 1-Cyan-1-(4'-pentyl-4-biphenylyl)-cyclohexan, F. 64°.

Analog erhält man aus den entsprechenden Acetonitrilen und den entsprechenden 1,5-Dibrompentanen:
r-1-Cyan-1-propyl-cis-4-p-methoxyphenyl-cyclohexan
r-1-Cyan-1-butyl-cis-4-p-methoxyphenyl-cyclohexan
r-1-Cyan-1-pentyl-cis-4-p-methoxyphenyl-cyclohexan
r-1-Cyan-1-hexyl-cis-4-p-methoxyphenyl-cyclohexan
r-1-Cyan-1-heptyl-cis-4-p-methoxyphenyl-cyclohexan
r-1-Cyan-1-propyl-cis-4-p-propylphenyl-cyclohexan
r-1-Cyan-1-butyl-cis-4-p-propylphenyl-cyclohexan
r-1-Cyan-1-pentyl-cis-4-p-propylphenyl-cyclohexan
r-1-Cyan-1-hexyl-cis-4-p-propylphenyl-cyclohexan
r-1-Cyan-1-heptyl-cis-4-p-propylphenyl-cyclohexan
r-1-Cyan-1-propyl-cis-4-p-butylphenyl-cyclohexan
r-1-Cyan-1-butyl-cis-4-p-butylphenyl-cyclohexan
r-1-Cyan-1-pentyl-cis-4-p-butylphenyl-cyclohexan
r-1-Cyan-1-hexyl-cis-4-p-butylphenyl-cyclohexan
r-1-Cyan-1-heptyl-cis-4-p-butylphenyl-cyclohexan
r-1-Cyan-1-propyl-cis-4-p-pentylphenyl-cyclohexan, K. -100°
r-1-Cyan-1-butyl-cis-4-p-pentylphenyl-cyclohexan
r-1-Cyan-1-pentyl-cis-4-p-pentylphenyl-cyclohexan
r-1-Cyan-1-hexyl-cis-4-p-pentylphenyl-cyclohexan
r-1-Cyan-1-heptyl-cis-4-p-pentylphenyl-cyclohexan.

**Beispiel 12**

Man löst 23,3 g trans,trans-4-Cyan-4'-propylcyclohexylcyclohexan und 41 g Butylbromid in 70 ml Toluol, versetzt mit 4,3 g $NaNH_2$ (50 % in Toluol) und kocht 5 Stunden. Nach üblicher Aufarbeitung erhält man r-1-Cyan-1-butyl-cis-4-(trans-4-propylcyclohexyl)-cyclohexan, F. 28°, K. 28,5°.

Analog erhält man durch Alkylierung entsprechender Nitrile:
r-1-Cyan-1-methyl-cis-4-(trans-4-ethylcyclohexyl)-cyclohexan
r-1-Cyan-1-methyl-cis-4-(trans-4-propylcyclohexyl)-cyclohexan
r-1-Cyan-1-methyl-cis-4-(trans-4-butylcyclohexyl)-cyclohexan
r-1-Cyan-1-methyl-cis-4-(trans-4-pentylcyclohexyl)-cyclohexan
r-1-Cyan-1-methyl-cis-4-(trans-4-hexylcyclohexyl)-cyclohexan
r-1-Cyan-1-methyl-cis-4-(trans-4-heptylcyclohexyl)-cyclohexan
r-1-Cyan-1-ethyl-cis-4-(trans-4-ethylcyclohexyl)-cyclohexan, F. 41°
r-1-Cyan-1-ethyl-cis-4-(trans-4-propylcyclohexyl)-cyclohexan
r-1-Cyan-1-ethyl-cis-4-(trans-4-butylcyclohexyl)-cyclohexan
r-1-Cyan-1-ethyl-cis-4-(trans-4-pentylcyclohexyl)-cyclohexan
r-1-Cyan-1-ethyl-cis-4-(trans-4-hexylcyclohexyl)-cyclohexan
r-1-Cyan-1-ethyl-cis-4-(trans-4-heptylcyclohexyl)-cyclohexan, F. 41°, K. -30°
r-1-Cyan-1-propyl-cis-4-(trans-4-methylcyclohexyl)-cyclohexan
r-1-Cyan-1-propyl-cis-4-(trans-4-ethylcyclohexyl)-cyclohexan
r-1-Cyan-1-propyl-cis-4-(trans-4-propylcyclohexyl)-cyclohexan, F. 32°, K. 13°
r-1-Cyan-1-propyl-cis-4-(trans-4-butylcyclohexyl)-cyclohexan, F. 31°, K. 27°
r-1-Cyan-1-propyl-cis-4-(trans-4-pentylcyclohexyl)-cyclohexan, F. 32°, K. 42°
r-1-Cyan-1-propyl-cis-4-(trans-4-hexylcyclohexyl)-cyclohexan
r-1-Cyan-1-propyl-cis-4-(trans-4-heptylcyclohexyl)-cyclohexan, F. 38°, K. 49°
r-1-Cyan-1-propyl-cis-4-(trans-4-octylcyclohexyl)-cyclohexan
r-1-Cyan-1-propyl-cis-4-(trans-4-nonylcyclohexyl)-cyclohexan
r-1-Cyan-1-propyl-cis-4-(trans-4-decylcyclohexyl)-cyclohexan
r-1-Cyan-1-isopropyl-cis-4-(trans-4-heptylcyclohexyl)-cyclohexan,F. 73°, K. -30°
r-1-Cyan-1-butyl-cis-4-(trans-4-methylcyclohexyl)-cyclohexan
r-1-Cyan-1-butyl-cis-4-(trans-4-ethylcyclohexyl)-cyclohexan
r-1-Cyan-1-butyl-cis-4-(trans-4-butylcyclohexyl)-cyclohexan, F. 9°, K. 36°

r-1-Cyan-1-butyl-cis-4-(trans-4-pentylcyclohexyl)-cyclohexan
r-1-Cyan-1-butyl-cis-4-(trans-4-hexylcyclohexyl)-cyclohexan
r-1-Cyan-1-butyl-cis-4-(trans-4-heptylcyclohexyl)-cyclohexan
r-1-Cyan-1-butyl-cis-4-(trans-4-octylcyclohexyl)-cyclohexan
r-1-Cyan-1-butyl-cis-4-(trans-4-nonylcyclohexyl)-cyclohexan
r-1-Cyan-1-butyl-cis-4-(trans-4-decylcyclohexyl)-cyclohexan
r-1-Cyan-1-pentyl-cis-4-(trans-4-methylcyclohexyl)-cyclohexan
r-1-Cyan-1-pentyl-cis-4-(trans-4-ethylcyclohexyl)-cyclohexan
r-1-Cyan-1-pentyl-cis-4-(trans-4-propylcyclohexyl)-cyclohexan, F. 39,5°, K. 48,5°
r-1-Cyan-1-pentyl-cis-4-(trans-4-butylcyclohexyl)-cyclohexan, F. -8°, K. 56°
r-1-Cyan-1-butyl-cis-4-(trans-4-pentylcyclohexyl)-cyclohexan, F. 28°, K. 63°
r-1-Cyan-1-butyl-cis-4-(trans-4-hexylcyclohexyl)-cyclohexan
r-1-Cyan-1-butyl-cis-4-(trans-4-heptylcyclohexyl)-cyclohexan, K. 56°
r-1-Cyan-1-butyl-cis-4-(trans-4-octylcyclohexyl)-cyclohexan
r-1-Cyan-1-butyl-cis-4-(trans-4-nonylcyclohexyl)-cyclohexan
r-1-Cyan-1-butyl-cis-4-(trans-4-decylcyclohexyl)-cyclohexan
r-1-Cyan-1-hexyl-cis-4-(trans-4-methylcyclohexyl)-cyclohexan
r-1-Cyan-1-hexyl-cis-4-(trans-4-ethylcyclohexyl)-cyclohexan
r-1-Cyan-1-hexyl-cis-4-(trans-4-propylcyclohexyl)-cyclohexan, F. 17°, K. 55°
r-1-Cyan-1-hexyl-cis-4-(trans-4-butylcyclohexyl)-cyclohexan, F. 30°, K. 55°
r-1-Cyan-1-hexl-cis-4-(trans-4-pentylcyclohexyl)-cyclohexan
r-1-Cyan-1-hexyl-cis-4-(trans-4-hexylcyclohexyl)-cyclohexan
r-1-Cyan-1-hexyl-cis-4-(trans-4-heptylcyclohexyl)-cyclohexan
r-1-Cyan-1-hexyl-cis-4-(trans-4-octylcyclohexyl)-cyclohexan
r-1-Cyan-1-hexyl-cis-4-(trans-4-nonylcyclohexyl)-cyclohexan
r-1-Cyan-1-hexyl-cis-4-(trans-4-decylcyclohexyl)-cyclohexan
r-1-Cyan-1-heptyl-cis-4-(trans-4-methylcyclohexyl)-cyclohexan
r-1-Cyan-1-heptyl-cis-4-(trans-4-ethylcyclohexyl)-cyclohexan
r-1-Cyan-1-heptyl-cis-4-(trans-4-propylcyclohexyl)-cyclohexan, F. 22°, K. 56°
r-1-Cyan-1-heptyl-cis-4-(trans-4-butylcyclohexyl)-cyclohexan, F. 28°, K. 61°
r-1-Cyan-1-heptyl-cis-4-(trans-4-pentylcyclohexyl)-cyclohexan, F. 50°
r-1-Cyan-1-heptyl-cis-4-(trans-4-hexylcyclohexyl)-cyclohexan
r-1-Cyan-1-heptyl-cis-4-(trans-4-heptylcyclohexyl)-cyclohexan
r-1-Cyan-1-heptyl-cis-4-(trans-4-octylcyclohexyl)-cyclohexan
r-1-Cyan-1-heptyl-cis-4-(trans-4-nonylcyclohexyl)-cyclohexan
r-1-Cyan-1-heptyl-cis-4-(trans-4-decylcyclohexyl)-cyclohexan
r-1-Cyan-1-octyl-cis-4-(trans-4-ethylcyclohexyl)-cyclohexan
r-1-Cyan-1-octyl-cis-4-(trans-4-propylcyclohexyl)-cyclohexan, K. 50°
r-1-Cyan-1-octyl-cis-4-(trans-4-butylcyclohexyl)-cyclohexan
r-1-Cyan-1-octyl-cis-4-(trans-4-pentylcyclohexyl)-cyclohexan
r-1-Cyan-1-octyl-cis-4-(trans-4-hexylcyclohexyl)-cyclohexan
r-1-Cyan-1-octyl-cis-4-(trans-4-heptylcyclohexyl)-cyclohexan
r-1-Cyan-1-nonyl-cis-4-(trans-4-ethylcyclohexyl)-cyclohexan
r-1-Cyan-1-nonyl-cis-4-(trans-4-propylcyclohexyl)-cyclohexan, K. 49°
r-1-Cyan-1-nonyl-cis-4-(trans-4-butylcyclohexyl)-cyclohexan
r-1-Cyan-1-nonyl-cis-4-(trans-4-pentylcyclohexyl)-cyclohexan
r-1-Cyan-1-nonyl-cis-4-(trans-4-hexylcyclohexyl)-cyclohexan
r-1-Cyan-1-nonyl-cis-4-(trans-4-heptylcyclohexyl)-cyclohexan
r-1-Cyan-1-decyl-cis-4-(trans-4-ethylcyclohexyl)-cyclohexan
r-1-Cyan-1-decyl-cis-4-(trans-4-propylcyclohexyl)-cyclohexan, F. 48°, K. 46°
r-1-Cyan-1-decyl-cis-4-(trans-4-butylcyclohexyl)-cyclohexan
r-1-Cyan-1-decyl-cis-4-(trans-4-pentylcyclohexyl)-cyclohexan
r-1-Cyan-1-decyl-cis-4-(trans-4-hexylcyclohexyl)-cyclohexan
r-1-Cyan-1-decyl-cis-4-(trans-4-heptylcyclohexyl)-cyclohexan
r-1-Cyan-1-methoxymethyl-cis-4-(trans-4-ethylcyclohexyl)-cyclohexan
r-1-Cyan-1-methoxymethyl-cis-4-(trans-4-propylcyclohexyl)-cyclohexan
r-1-Cyan-1-methoxymethyl-cis-4-(trans-4-butylcyclohexyl)-cyclohexan
r-1-Cyan-1-methoxymethyl-cis-4-(trans-4-pentylcyclohexyl)-cyclohexan

r-1-Cyan-1-methoxymethyl-cis-4-(trans-4-hexylcyclohexyl)-cyclohexan
r-1-Cyan-1-methoxymethyl-cis-4-(trans-4-heptylcyclohexyl)-cyclohexan
r-1-Cyan-1-ethoxymethyl-cis-4-(trans-4-pentylcycloheyl)-cyclohexan, K. -50 °
r-1-Cyan-1-(2,5-dioxahexyl)-cis-4-(trans-4-pentylcyclohexyl)-cyclohexan
r-1-Cyan-1-pentyl-2,2-difluor-cis-4-(trans-4-pentylcyclohexyl)-cyclohexan
r-1-Cyan-1-pentyl-cis-4-p-methoxyphenyl-cyclohexan
r-1-Cyan-1-propyl-cis-4-[2-(trans-4-propylcyclohexyl)-ethyl]-cyclohexan
r-1-Cyan-1-propyl-cis-4-[2-(trans-4-butylcyclohexyl)-ethyl]-cyclohexan
r-1-Cyan-1-propyl-cis-4-[2-(trans-4-pentylcyclohexyl)-ethyl]-cyclohexan
r-1-Cyan-1-propyl-cis-4-[2-(trans-4-hexylcyclohexyl)-ethyl]-cyclohexan
r-1-Cyan-1-propyl-cis-4-[2-(trans-4-heptylcyclohexyl)-ethyl]-cyclohexan
r-1-Cyan-1-butyl-cis-4-[2-(trans-4-propylcyclohexyl)-ethyl]-cyclohexan
r-1-Cyan-1-butyl-cis-4-[2-(trans-4-butylcyclohexyl)-ethyl]-cyclohexan
r-1-Cyan-1-butyl-cis-4-[2-(trans-4-pentylcyclohexyl)-ethyl]-cyclohexan
r-1-Cyan-1-butyl-cis-4-[2-(trans-4-hexylcyclohexyl)-ethyl]-cyclohexan
r-1-Cyan-1-butyl-cis-4-[2-(trans-4-heptylcyclohexyl)-ethyl]-cyclohexan
r-1-Cyan-1-pentyl-cis-4-[2-(trans-4-propylcyclohexyl)-ethyl]-cyclohexan
r-1-Cyan-1-pentyl-cis-4-[2-(trans-4-butylcyclohexyl)-ethyl]-cyclohexan
r-1-Cyan-1-pentyl-cis-4-[2-(trans-4-pentylcyclohexyl)-ethyl]-cyclohexan
r-1-Cyan-1-pentyl-cis-4-[2-(trans-4-hexylcyclohexyl)-ethyl]-cyclohexan
r-1-Cyan-1-pentyl-cis-4-[2-(trans-4-heptylcyclohexyl)-ethyl]-cyclohexan
r-1-Cyan-1-hexyl-cis-4-[2-(trans-4-propylcyclohexyl)-ethyl]-cyclohexan
r-1-Cyan-1-hexyl-cis-4-[2-(trans-4-butylcyclohexyl)-ethyl]-cyclohexan
r-1-Cyan-1-hexyl-cis-4-[2-(trans-4-pentylcyclohexyl)-ethyl]-cyclohexan
r-1-Cyan-1-hexyl-cis-4-[2-(trans-4-hexylcyclohexyl)-ethyl]-cyclohexan
r-1-Cyan-1-hexyl-cis-4-[2-(trans-4-heptylcyclohexyl)-ethyl]-cyclohexan
r-1-Cyan-1-heptyl-cis-4-[2-(trans-4-propylcyclohexyl)-ethyl]-cyclohexan
r-1-Cyan-1-heptyl-cis-4-[2-(trans-4-butylcyclohexyl)-ethyl]-cyclohexan
r-1-Cyan-1-heptyl-cis-4-[2-(trans-4-pentylcyclohexyl)-ethyl]-cyclohexan
r-1-Cyan-1-heptyl-cis-4-[2-(trans-4-hexylcyclohexyl)-ethyl]-cyclohexan
r-1-Cyan-1-heptyl-cis-4-[2-(trans-4-heptylcyclohexyl)-ethyl]-cyclohexan
r-1-Cyan-1-propyl-cis-4-[2-(trans-4-(trans-4-propylcyclohexyl)-cyclohexyl)-ethyl]-cyclohexan
r-1-Cyan-1-propyl-cis-4-[2-(trans-4-(trans-4-pentylcyclohexyl)-cyclohexyl)-ethyl]-cyclohexan
r-1-Cyan-1-pentyl-cis-4-[2-(trans-4-(trans-4-propylcyclohexyl)-cyclohexyl)-ethyl]-cyclohexan
r-1-Cyan-1-pentyl-cis-4-[2-(trans-4-(trans-4-pentylcyclohexyl)-cyclohexyl)-ethyl]-cyclohexan
r-1-Cyan-1-[2-(trans-4-(trans-4-propylcyclohexyl)-cyclohexyl)-ethyl]-cis-4-propyl-cyclohexan
r-1-Cyan-1-[2-(trans-4-(trans-4-propylcyclohexyl)-cyclohexyl)-ethyl]-cis-4-pentyl-cyclohexan
r-1-Cyan-1-propyl-cis-4-(trans-4-propylcyclohexyl-methoxy)-cyclohexan
r-1-Cyan-1-propyl-cis-4-(trans-4-butylcyclohexyl-methoxy)-cyclohexan
r-1-Cyan-1-propyl-cis-4-(trans-4-pentylcyclohexyl-methoxy)-cyclohexan
r-1-Cyan-1-propyl-cis-4-(trans-4-hexylcyclohexyl-methoxy)-cyclohexan
r-1-Cyan-1-propyl-cis-4-(trans-4-heptylcyclohexyl-methoxy)-cyclohexan
r-1-Cyan-1-butyl-cis-4-(trans-4-propylcyclohexyl-methoxy)-cyclohexan
r-1-Cyan-1-butyl-cis-4-(trans-4-butylcyclohexyl-methoxy)-cyclohexan
r-1-Cyan-1-butyl-cis-4-(trans-4-pentylcyclohexyl-methoxy)-cyclohexan
r-1-Cyan-1-butyl-cis-4-(trans-4-hexylcyclohexyl-methoxy)-cyclohexan
r-1-Cyan-1-butyl-cis-4-(trans-4-heptylcyclohexyl-methoxy)-cyclohexan
r-1-Cyan-1-pentyl-cis-4-(trans-4-propylcyclohexyl-methoxy)-cyclohexan
r-1-Cyan-1-pentyl-cis-4-(trans-4-butylcyclohexyl-methoxy)-cyclohexan
r-1-Cyan-1-pentyl-cis-4-(trans-4-pentylcyclohexyl-methoxy)-cyclohexan
r-1-Cyan-1-pentyl-cis-4-(trans-4-hexylcyclohexyl-methoxy)-cyclohexan
r-1-Cyan-1-pentyl-cis-4-(trans-4-heptylcyclohexyl-methoxy)-cyclohexan
r-1-Cyan-1-hexyl-cis-4-(trans-4-propylcyclohexyl-methoxy)-cyclohexan
r-1-Cyan-1-hexyl-cis-4-(trans-4-butylcyclohexyl-methoxy)-cyclohexan
r-1-Cyan-1-hexyl-cis-4-(trans-4-pentylcyclohexyl-methoxy)-cyclohexan
r-1-Cyan-1-hexyl-cis-4-(trans-4-hexylcyclohexyl-methoxy)-cyclohexan
r-1-Cyan-1-hexyl-cis-4-(trans-4-heptylcyclohexyl-methoxy)-cyclohexan
r-1-Cyan-1-heptyl-cis-4-(trans-4-propylcyclohexyl-methoxy)-cyclohexan

r-1-Cyan-1-heptyl-cis-4-(trans-4-butylcyclohexyl-methoxy)-cyclohexan
r-1-Cyan-1-heptyl-cis-4-(trans-4-pentylcyclohexyl-methoxy)-cyclohexan
r-1-Cyan-1-heptyl-cis-4-(trans-4-hexylcyclohexyl-methoxy)-cyclohexan
r-1-Cyan-1-heptyl-cis-4-(trans-4-heptylcyclohexyl-methoxy)-cyclohexan
r-1-Cyan-1-propyl-cis-4-(trans-4-propylcyclohexoxy methoxy)-cyclohexan
r-1-Cyan-1-propyl-cis-4-(trans-4-butylcyclohexoxy methoxy)-cyclohexan
r-1-Cyan-1-propyl-cis-4-(trans-4-pentylcyclohexoxy methoxy)-cyclohexan
r-1-Cyan-1-propyl-cis-4-(trans-4-hexylcyclohexoxy methoxy)-cyclohexan
r-1-Cyan-1-propyl-cis-4-(trans-4-heptylcyclohexoxy methoxy)-cyclohexan
r-1-Cyan-1-butyl-cis-4-(trans-4-propylcyclohexoxy methoxy)-cyclohexan
r-1-Cyan-1-butyl-cis-4-(trans-4-butylcyclohexoxy methoxy)-cyclohexan
r-1-Cyan-1-butyl-cis-4-(trans-4-pentylcyclohexoxy methoxy)-cyclohexan
r-1-Cyan-1-butyl-cis-4-(trans-4-hexylcyclohexoxy methoxy)-cyclohexan
r-1-Cyan-1-butyl-cis-4-(trans-4-heptylcyclohexoxy methoxy)-cyclohexan
r-1-Cyan-1-pentyl-cis-4-(trans-4-propylcyclohexoxy methoxy)-cyclohexan
r-1-Cyan-1-pentyl-cis-4-(trans-4-butylcyclohexoxy methoxy)-cyclohexan
r-1-Cyan-1-pentyl-cis-4-(trans-4-pentylcyclohexoxy methoxy)-cyclohexan
r-1-Cyan-1-pentyl-cis-4-(trans-4-hexylcyclohexoxy methoxy)-cyclohexan
r-1-Cyan-1-pentyl-cis-4-(trans-4-heptylcyclohexoxy methoxy)-cyclohexan
r-1-Cyan-1-hexyl-cis-4-(trans-4-propylcyclohexoxy methoxy)-cyclohexan
r-1-Cyan-1-hexyl-cis-4-(trans-4-butylcyclohexoxy methoxy)-cyclohexan
r-1-Cyan-1-hexyl-cis-4-(trans-4-pentylcyclohexoxy methoxy)-cyclohexan
r-1-Cyan-1-hexyl-cis-4-(trans-4-hexylcyclohexoxy methoxy)-cyclohexan
r-1-Cyan-1-hexyl-cis-4-(trans-4-heptylcyclohexoxy methoxy)-cyclohexan
r-1-Cyan-1-heptyl-cis-4-(trans-4-propylcyclohexoxy methoxy)-cyclohexan
r-1-Cyan-1-heptyl-cis-4-(trans-4-butylcyclohexoxy methoxy)-cyclohexan
r-1-Cyan-1-heptyl-cis-4-(trans-4-pentylcyclohexoxy methoxy)-cyclohexan
r-1-Cyan-1-heptyl-cis-4-(trans-4-hexylcyclohexoxy methoxy)-cyclohexan
r-1-Cyan-1-heptyl-cis-4-(trans-4-heptylcyclohexoxy methoxy)-cyclohexan

**Beispiel 13**

Eine Lösung von 29,4 g r-1-Hydroxy-1-pentyl-cis-4-(trans-4-propylcyclohexyl)-cyclohexan [erhältlich durch Oxidation von 4-(trans-4-Propylcyclohexyl)-cyclohexanol mit $CrO_3$ zum Keton, Reaktion mit Pentyl-MgBr und Hydrolyse] in 280 ml 1,2-Dimethoxyethan wird mit 4,8 g NaH und 27,8 g $CH_3J$ versetzt. Nach 5 Std. Erhitzen auf 70° kühlt man ab, zersetzt mit Wasser, arbeitet wie üblich auf und erhält r-1-Methoxy-1-pentyl-cis-4-(trans-4-propylcyclohexyl)-cyclohexan, F. 43°, K. 135°.

Analog erhält man durch Veretherung:

r-1-Methoxy-1-propyl-cis-4-(trans-4-propylcyclohexyl)-cyclohexan
r-1-Methoxy-1-propyl-cis-4-(trans-4-butylcyclohexyl)-cyclohexan
r-1-Methoxy-1-propyl-cis-4-(trans-4-pentylcyclohexyl)-cyclohexan
r-1-Methoxy-1-propyl-cis-4-(trans-4-hexylcyclohexyl)-cyclohexan
r-1-Methoxy-1-propyl-cis-4-(trans-4-heptylcyclohexyl)-cyclohexan
r-1-Methoxy-1-butyl-cis-4-(trans-4-propylcyclohexyl)-cyclohexan
r-1-Methoxy-1-butyl-cis-4-(trans-4-butylcyclohexyl)-cyclohexan
r-1-Methoxy-1-butyl-cis-4-(trans-4-pentylcyclohexyl)-cyclohexan
r-1-Methoxy-1-butyl-cis-4-(trans-4-hexylcyclohexyl)-cyclohexan
r-1-Methoxy-1-butyl-cis-4-(trans-4-heptylcyclohexyl)-cyclohexan
r-1-Methoxy-1-pentyl-cis-4-(trans-4-butylcyclohexyl)-cyclohexan
r-1-Methoxy-1-pentyl-cis-4-(trans-4-pentylcyclohexyl)-cyclohexan
r-1-Methoxy-1-pentyl-cis-4-(trans-4-hexylcyclohexyl)-cyclohexan
r-1-Methoxy-1-pentyl-cis-4-(trans-4-heptylcyclohexyl)-cyclohexan
r-1-Methoxy-1-hexyl-cis-4-(trans-4-propylcyclohexyl)-cyclohexan
r-1-Methoxy-1-hexyl-cis-4-(trans-4-butylcyclohexyl)-cyclohexan
r-1-Methoxy-1-hexyl-cis-4-(trans-4-pentylcyclohexyl)-cyclohexan
r-1-Methoxy-1-hexyl-cis-4-(trans-4-hexylcyclohexyl)-cyclohexan
r-1-Methoxy-1-hexyl-cis-4-(trans-4-heptylcyclohexyl)-cyclohexan
r-1-Methoxy-1-heptyl-cis-4-(trans-4-propylcyclohexyl)-cyclohexan

r-1-Methoxy-1-heptyl-cis-4-(trans-4-butylcyclohexyl)-cyclohexan
r-1-Methoxy-1-heptyl-cis-4-(trans-4-pentylcyclohexyl)-cyclohexan
r-1-Methoxy-1-heptyl-cis-4-(trans-4-hexylcyclohexyl)-cyclohexan
r-1-Methoxy-1-heptyl-cis-4-(trans-4-heptylcyclohexyl)-cyclohexan
r-1-Ethoxy-1-propyl-cis-4-(trans-4-propylcyclohexyl)-cyclohexan
r-1-Ethoxy-1-propyl-cis-4-(trans-4-butylcyclohexyl)-cyclohexan
r-1-Ethoxy-1-propyl-cis-4-(trans-4-pentylcyclohexyl)-cyclohexan
r-1-Ethoxy-1-propyl-cis-4-(trans-4-hexylcyclohexyl)-cyclohexan
r-1-Ethoxy-1-propyl-cis-4-(trans-4-heptylcyclohexyl)-cyclohexan
r-1-Ethoxy-1-butyl-cis-4-(trans-4-propylcyclohexyl)-cyclohexan
r-1-Ethoxy-1-butyl-cis-4-(trans-4-butylcyclohexyl)-cyclohexan
r-1-Ethoxy-1-butyl-cis-4-(trans-4-pentylcyclohexyl)-cyclohexan
r-1-Ethoxy-1-butyl-cis-4-(trans-4-hexylcyclohexyl)-cyclohexan
r-1-Ethoxy-1-butyl-cis-4-(trans-4-heptylcyclohexyl)-cyclohexan
r-1-Ethoxy-1-pentyl-cis-4-(trans-4-propylcyclohexyl)-cyclohexan
r-1-Ethoxy-1-pentyl-cis-4-(trans-4-butylcyclohexyl)-cyclohexan
r-1-Ethoxy-1-pentyl-cis-4-(trans-4-pentylcyclohexyl)-cyclohexan
r-1-Ethoxy-1-pentyl-cis-4-(trans-4-hexylcyclohexyl)-cyclohexan
r-1-Ethoxy-1-pentyl-cis-4-(trans-4-heptylcyclohexyl)-cyclohexan
r-1-Ethoxy-1-hexyl-cis-4-(trans-4-propylcyclohexyl)-cyclohexan
r-1-Ethoxy-1-hexyl-cis-4-(trans-4-butylcyclohexyl)-cyclohexan
r-1-Ethoxy-1-hexyl-cis-4-(trans-4-pentylcyclohexyl)-cyclohexan
r-1-Ethoxy-1-hexyl-cis-4-(trans-4-hexylcyclohexyl)-cyclohexan
r-1-Ethoxy-1-hexyl-cis-4-(trans-4-heptylcyclohexyl)-cyclohexan
r-1-Ethoxy-1-heptyl-cis-4-(trans-4-propylcyclohexyl)-cyclohexan
r-1-Ethoxy-1-heptyl-cis-4-(trans-4-butylcyclohexyl)-cyclohexan
r-1-Ethoxy-1-heptyl-cis-4-(trans-4-pentylcyclohexyl)-cyclohexan
r-1-Ethoxy-1-heptyl-cis-4-(trans-4-hexylcyclohexyl)-cyclohexan
r-1-Ethoxy-1-heptyl-cis-4-(trans-4-heptylcyclohexyl)-cyclohexan

**Beispiel 14**

Ein Gemisch aus 30,6 g r-1-Cyan-1-p-bromphenyl-cis-4-propylcyclohexan, 10 g $Cu_2(CN)_2$, 120 ml Pyridin und 60 ml N-Methylpyrrolidon wird 2 Std. auf 150° erhitzt. Man kühlt ab, gibt eine Lösung von 120 g $FeCl_3 \cdot 6\ H_2O$ in 600 ml 20%iger Salzsäure hinzu, erwärmt 1,5 Std. unter Rühren auf 70°, arbeitet wie üblich auf und erhält r-1-Cyan-1-p-cyanphenyl-cis-4-propylcyclohexan.

**Beispiel 15**

Ein Gemisch aus 29,4 g 1-Propyl-cis-4-(trans-4-propylcyclohexyl)-cyclohexan-r-1-carbonsäure (erhältlich durch Hydrolyse des Nitrils), 21,6 g $SF_4$ und 300 ml Cyclohexan wird in einem Hastelloy-Autoklaven 24 Std. auf 150° erhitzt. Nach dem Abkühlen wird entspannt, evakuiert, mehrfach mit Stickstoff belüftet und das Reaktionsgemisch auf Eis gegossen. Nach üblicher Aufarbeitung erhält man r-1-Trifluormethyl-1-propyl-cis-4-(trans-4-propylcyclohexyl)-cyclohexan.

Analog sind aus den entsprechenden Carbonsäuren erhältlich
r-1-Trifluormethyl-1-propyl-cis-4-(trans-4-butylcyclohexyl)-cyclohexan
r-1-Trifluormethyl-1-propyl-cis-4-(trans-4-pentylcyclohexyl)-cyclohexan
r-1-Trifluormethyl-1-propyl-cis-4-(trans-4-hexylcyclohexyl)-cyclohexan
r-1-Trifluormethyl-1-propyl-cis-4-(trans-4-heptylcyclohexyl)-cyclohexan
r-1-Trifluormethyl-1-butyl-cis-4-(trans-4-propylcyclohexyl)-cyclohexan
r-1-Trifluormethyl-1-butyl-cis-4-(trans-4-butylcyclohexyl)-cyclohexan
r-1-Trifluormethyl-1-butyl-cis-4-(trans-4-pentylcyclohexyl)-cyclohexan
r-1-Trifluormethyl-1-butyl-cis-4-(trans-4-hexylcyclohexyl)-cyclohexan
r-1-Trifluormethyl-1-butyl-cis-4-(trans-4-heptylcyclohexyl)-cyclohexan
r-1-Trifluormethyl-1-pentyl-cis-4-(trans-4-propylcyclohexyl)-cyclohexan
r-1-Trifluormethyl-1-pentyl-cis-4-(trans-4-butylcyclohexyl)-cyclohexan
r-1-Trifluormethyl-1-pentyl-cis-4-(trans-4-pentylcyclohexyl)-cyclohexan
r-1-Trifluormethyl-1-pentyl-cis-4-(trans-4-hexylcyclohexyl)-cyclohexan

r-1-Trifluormethyl-1-pentyl-cis-4-(trans-4-heptylcyclohexyl)-cyclohexan
r-1-Trifluormethyl-1-hexyl-cis-4-(trans-4-propylcyclohexyl)-cyclohexan
r-1-Trifluormethyl-1-hexyl-cis-4-(trans-4-butylcyclohexyl)-cyclohexan
r-1-Trifluormethyl-1-hexyl-cis-4-(trans-4-pentylcyclohexyl)-cyclohexan
r-1-Trifluormethyl-1-hexyl-cis-4-(tans-4-hexylcyclohexyl)-cyclohexan
r-1-Trifluormethyl-1-hexyl-cis-4-(trans-4-heptylcyclohexyl)-cyclohexan
r-1-Trifluormethyl-1-heptyl-cis-4-(trans-4-propylcyclohexyl)-cyclohexan
r-1-Trifluormethyl-1-heptyl-cis-4-(trans-4-butylcyclohexyl)-cyclohexan
r-1-Trifluormethyl-1-heptyl-cis-4-(trans-4-pentylcyclohexyl)-cyclohexan
r-1-Trifluormethyl-1-heptyl-cis-4-(trans-4-hexylcyclohexyl)-cyclohexan
r-1-Trifluormethyl-1-heptyl-cis-4-(trans-4-heptylcyclohexyl)-cyclohexan

Es folgen Beispiele für erfindungsgemäße Dielektrika mit einem Gehalt an mindestens einer Verbindung der Formel I:

Beispiel A

Ein Gemisch aus

| | |
|---|---|
| 11 % | r-1-Cyan-1-pentyl-cis-4-(trans-4-propylcyclohexyl)-cyclohexan |
| 24 % | r-1-Cyan-1-heptyl-cis-4-(trans-4-propylcyclohexyl)-cyclohexan |
| 21 % | r-1-Cyan-1-propyl-cis-4-(trans-4-pentylcyclohexyl)-cyclohexan |
| 21 % | r-1-Cyan-1-pentyl-cis-4-(trans-4-pentylcyclohexyl)-cyclohexan |
| 13 % | r-1-Cyan-1-propyl-cis-4-(trans-4-heptylcyclohexyl)-cyclohexan |
| 10 % | 4-(trans-4-Pentylcyclohexyl)-4'-(trans-4-propylcyclohexyl)-biphenyl |

zeigt F. -11°, K. 75°.

**Beispiel B**

In 98 Gewichtsteilen des Gemisches nach Beispiel A löst man 2 Gewichtsteile des blauen Farbstoffs 4,8-Diamino-1,5-dihydroxy-2-p-methoxyphenyl-anthrachinon. Ordnungsgrad des Farbstoffs 0,71.

**Beispiel C**

Ein Gemisch aus

| | |
|---|---|
| 9 % | r-1-Cyan-1-pentyl-cis-4-(trans-4-propylcyclohexyl)-cyclohexan |
| 19 % | r-1-Cyan-1-heptyl-cis-4-(trans-4-propylcyclohexyl)-cyclohexan |
| 17 % | r-1-Cyan-1-propyl-cis-4-(trans-4-pentylcyclohexyl)-cyclohexan |
| 17 % | r-1-Cyan-1-pentyl-cis-4-(trans-4-pentylcyclohexyl)-cyclohexan |
| 10 % | r-1-Cyan-1-propyl-cis-4-(trans-4-heptylcyclohexyl)-cyclohexan |
| 28 % | 4-Ethyl-2'-fluor-4'-(trans-4-pentylcyclohexyl)-biphenyl |

zeigt F. -16°, K. 66°.

**Beispiel D**

In 98 Gewichtsteilen des Gemisches nach Beispiel C löst man 2 Gewichtsteile des roten Farbstoffs 1-p-Dimethylaminobenzylidenamino-4-p-cyanphenylazonaphthalin. Ordnungsgrad des Farbstoffs 0,68.

**Beispiel E**

Ein Gemisch aus

| | |
|---|---|
| 13 % | r-1-Cyan-1-pentyl-cis-4-(trans-4-propylcyclohexyl)-cyclohexan |
| 27 % | r-1-Cyan-1-heptyl-cis-4-(trans-4-propylcyclohexyl)-cyclohexan |
| 23 % | r-1-Cyan-1-propyl-cis-4-(trans-4-pentylcyclohexyl)-cyclohexan |
| 23 % | r-1-Cyan-1-pentyl-cis-4-(trans-4-pentylcyclohexyl)-cyclohexan |
| 14 % | r-1-Cyan-1-propyl-cis-4-(trans-4-heptylcyclohexyl)-cyclohexan |

zeigt F. -9°, K. 52°.

**Beispiel F**

In 99 Gewichtsteilen des Gemisches nach Beispiel E löst man 1 Gewichtsteil Perylen-3,9-bis-carbonsäure-bis-(p-isopropylphenylester).Ordnungsgrad des Farbstoffs 0,73.

**Beispiel G**

Ein Gemisch aus

| | |
|---|---|
| 12 % | r-1-Cyan-1-pentyl-cis-4-(trans-4-propylcyclohexyl)-cyclohexan |
| 25 % | r-1-Cyan-1-heptyl-cis-4-(trans-4-propylcyclohexyl)-cyclohexan |
| 22 % | r-1-Cyan-1-propyl-cis-4-(trans-4-pentylcyclohexyl)-cyclohexan |
| 22 % | r-1-Cyan-1-pentyl-cis-4-(trans-4-pentylcyclohexyl)-cyclohexan |
| 13 % | r-1-Cyan-1-propyl-cis-4-(trans-4-heptylcyclohexyl)-cyclohexan |
| 6 % | trans,trans-4-Propylcyclohexylcyclohexan-4'-carbonsäure-trans-(4-propylcyclohexylester) |

zeigt F. -10°, K. 59°.

**Beispiel H**

Ein Gemisch aus

| | |
|---|---|
| 12 % | r-1-Cyan-1-pentyl-cis-4-(trans-4-propylcyclohexyl)-cyclohexan |
| 24 % | r-1-Cyan-1-heptyl-cis-4-(trans-4-propylcyclohexyl)-cyclohexan |
| 21 % | r-1-Cyan-1-propyl-cis-4-(trans-4-pentylcyclohexyl)-cyclohexan |
| 21 % | r-1-Cyan-1-pentyl-cis-4-(trans-4-pentylcyclohexyl)-cyclohexan |
| 13 % | r-1-Cyan-1-propyl-cis-4-(trans-4-heptylcyclohexyl)-cyclohexan |
| 9 % | trans-4-Pentylcyclohexancarbonsäure-(p-trans-4-butylcyclohexylphenylester) |

zeigt F. -11°, K. 63°.

**Beispiel I**

Ein Gemisch aus

| | |
|---|---|
| 19 % | r-1-Cyan-1-heptyl-cis-4-(trans-4-propylcyclohexyl)-cylcohexan |
| 18 % | r-1-Cyan-1-pentyl-cis-4-(trans-4-pentylcyclohexyl)-cylcohexan |
| 11 % | trans-4-Propylcyclohexancarbonsäure-(p-ethoxyphenylester) |
| 9 | 4-(trans-4-Pentylcyclohexyl)-4'-(trans-4-propylcyclohexyl)-biphenyl |
| 28 % | 4-Ethyl-2'-fluor-4'-(trans-4-pentylcyclohexyl)-biphenyl |
| 15 % | trans-1-(p-Ethoxyphenyl)-4-propylcyclohexan |

zeigt F. -15°, K. 86°.

**Beispiel J**

Ein Gemisch aus

| | |
|---|---|
| 18 % | r-1-Cyan-1-heptyl-cis-4-(trans-4-propylcyclohexyl)-cyclohexan |
| 17 % | r-1-Cyan-1-propyl-cis-4-(trans-4-pentylcyclohexyl)-cyclohexan |
| 17 % | r-1-Cyan-1-pentyl-cis-4-(trans-4-pentylcyclohexyl)-cyclohexan |
| 10 % | r-1-Cyan-1-propyl-cis-4-(trans-4-heptylcyclohexyl)-cyclohexan |
| 24 % | p-(trans-4-Propylcyclohexyl)-benzoesäure-(4-butyl-2-cyanphenylester) |
| 14 % | trans-1-(p-Ethoxyphenyl)-4-propylcyclohexan |

zeigt F. -16°, K. 63°.

**Beispiel K**

Ein Gemisch aus

| | |
|---|---|
| 19 % | r-1-Cyan-1-pentyl-cis-4-(trans-4-propylcyclohexyl)-cyclohexan |
| 31 % | r-1-Cyan-1-propyl-cis-4-(trans-4-pentylcyclohexyl)-cyclohexan |
| 33 % | r-1-Cyan-1-pentyl-cis-4-(trans-4-pentylcyclohexyl)-cyclohexan |
| 17 % | 4-(trans-4-Pentylcyclohexyl)-4'-(trans-4-propylcyclohexyl)-biphenyl |

zeigt F. -2°, K. 91°.

**Beispiel L**

Ein Gemisch aus

| | |
|---|---|
| 11 % | r-1-Cyan-1-pentyl-cis-4-(trans-4-propylcyclohexyl)-cyclohexan |
| 25 % | r-1-Cyan-1-hepty-1-cis-4-(trans-4-propylcyclohexyl)-cyclohexan |
| 21 % | r-1-Cyan-1-propyl-cis-4-(trans-4-pentylcyclohexyl)-cyclohexan |
| 22 % | r-1-Cyan-1-pentyl-cis-4-(trans-4-pentylcyclohexyl)-cyclohexan |
| 13 % | r-1-Cyan-1-propyl-cis-4-(trans-4-heptylcyclohexyl)-cyclohexan |
| 8 % | p-(trans-4-Propylcyclohexyl)-benzoesäure-(trans-4-propylcyclohexylester) |

zeigt F. -11°, K. 60°.

**Beispiel M**

Ein Gemisch aus

| | |
|---|---|
| 22 % | r-1-Cyan-1-heptyl-cis-4-(trans-4-propylcyclohexyl)-cyclohexan |
| 20 % | r-1-Cyan-1-pentyl-cis-4-(trans-4-pentylcyclohexyl)-cyclohexan |
| 18 % | trans-4-Pentylcyclohexancarbonsäure-(trans-4-propylcyclohexylester) |
| 10 % | 4-(trans-4-Pentylcyclohexyl)-4'-(trans-4-propylcyclohexyl)-biphenyl |
| 30 % | 4-Ethyl-2'-fluor-4'-(trans-4-pentylcyclohexyl)-biphenyl |

zeigt F. -12°, K. 92°.

**Beispiel N**

Ein Gemisch aus

| | |
|---|---|
| 17 % | r-1-Cyan-1-pentyl-cis-4-(trans-4-pentylcyclohexyl)-cyclohexan |
| 43 % | trans-4-Propylcyclohexancarbonsäure-(trans-4-propylcyclohexylester) |
| 16 % | trans-4-Pentylcyclohexancarbonsäure-(trans-4-propylcyclohexylester) |
| 17 % | p-(trans-4-Propylcyclohexyl)-benzoesäure-(4-butyl-2-cyanphenylester) |

zeigt F. -16°, K. 58°.

**Beispiel O**

Ein Gemisch aus

| | |
|---|---|
| 9 % | 2-p-Cyanphenyl-5-propyl-1,3-dioxan |
| 12 % | 2-p-Cyanphenyl-5-butyl-1,3-dioxan |
| 9 % | 2-p-Cyanphenyl-5-pentyl-1,3-dioxan |
| 6 % | 2-p-Octoxyphenyl-5-pentyl-pyrimidin |
| 5 % | 2-p-Nonoxyphenyl-5-pentyl-pyrimidin |
| 5 % | 2-p-Heptoxyphenyl-5-hexyl-pyrimidin |
| 4 % | 2-p-Nonoxyphenyl-5-hexyl-pyrimidin |
| 6 % | 4,4'-Bis-(trans-4-propylcyclohexyl)-biphenyl |
| 9 % | 4-(trans-4-Pentylcyclohexyl)-4'-(trans-4-propylcyclohexyl)-biphenyl |
| 17 % | r-1-Cyan-1-pentyl-cis-4-(trans-4-propylcyclohexyl)-cyclohexan und |
| 18 % | r-1-Cyan-1-heptyl-cis-4-(trans-4-propylcyclohexyl)-cyclohexan |

zeigt F. -5°, K. 66°. Dieses Gemisch hat eine besonders steile Kennlinie.

## Patentansprüche

**1.** Cyclohexanderivate der Formel I

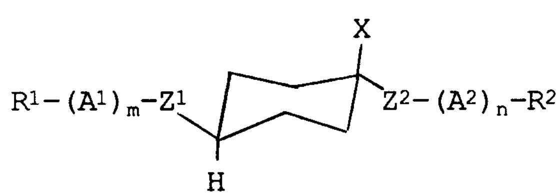

worin

| | |
|---|---|
| $R^1$ und $R^2$ | jeweils geradkettige oder höchstens einfach verzweigte Alkylgruppen mit 1-10 C-Atomen, worin auch eine oder zwei $CH_2$-Gruppen durch O-Atome ersetzt sein können, F, Cl, Br, CN oder -O-COR, |
| $A^1$ und $A^2$ | jeweils unsubstituierte oder durch 1-4 F-Atome substituierte 1,4-Phenylen-, 1,4-Cyclohexylen-, 1,3-Dioxan-2,5-diyl-, Piperidin-1,4-diyl, 1,4-Bicyclo(2,2,2)-octylen- oder Pyrimidin-2,5-diylgruppen, |
| x | Alkyl, Alkoxy, fluoriertes Alkyl oder fluoriertes Alkoxy mit jeweils 1-5 C-Atomen, F, Cl, Br oder CN, |
| $Z^1$ und $Z^2$ | jeweils -CO-O-, -O-CO-, $-CH_2CH_2-$, $-OCH_2-$, $-CH_2O-$ oder eine Einfachbindung, |
| R | eine Alkylgruppe mit 1-5 C-Atomen, |
| m | 1 oder 2 und |
| n | 0 oder 1 |

bedeuten,

wobei für m = 2 die beiden Gruppen $A^1$ gleich oder voneinander verschieden sein können, und die

Gruppen $R^1$-$(A^1)_m$-$Z^1$- und -$Z^2$-$(A^2)_n$-$R^2$ in trans-Stellung zueinander stehen, während der Substituent X in cis-Stellung zu der gegenüberstehenden Gruppe steht.

2. Cyclohexanderivate nach Anspruch 1, dadurch gekennzeichnet, daß X eine CN-, $CH_3$-, $CH_3O$- oder $CF_3$-Gruppe bedeutet.

3. Cyclohexanderivate nach Anspruch 1 oder 2, dadurch gekennzeichnet, daß $A^1$ und $A^2$ jeweils 1,4-Cyclohexylen oder unsubstituiertes oder durch 1-4 Fluoratome substituiertes 1,4-Phenylen bedeutet.

4. Cyclohexanderivate nach mindestens einem der Ansprüche 1 bis 3, dadurch gekennzeichnet, daß $Z^1$ und $Z^2$ Einfachbindungen bedeuten.

5. Cyclohexanderivate nach mindestens einem der Ansprüche 1 bis 4, dadurch gekennzeichnet, daß n 0 bedeutet.

6. Cyclohexanderivate nach mindestens einem der Ansprüche 1 bis 5, dadurch gekennzeichnet, daß $R^1$ und $R^2$ Ethyl, Propyl, Butyl, Pentyl, Hexyl, Heptyl, Octyl, Nonyl, Decyl, Ethoxy, Propoxy, Butoxy, Pentoxy, Hexoxy, Heptoxy, Octoxy, Nonoxy, Decoxy, 2-Oxapropyl (= Methoxymethyl), 2- (= Ethoxethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl, Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl oder 3-Oxa-4-methylpentyl bedeuten.

7. Cyclohexanderivate nach mindestens einem der Ansprüche 1 bis 6, dadurch gekennzeichnet, daß sie den Formeln Iaj oder Iat entsprechen,

$R^1$-Cy-A-$R^2$ Iaj

$R^1$-Phe-Phe-A-$R^2$ Iat

worin Cy 1,4-Cyclohexylen, Phe unsubstituiertes oder durch 1-4-Fluoratome substituiertes 1,4-Phenylen und A

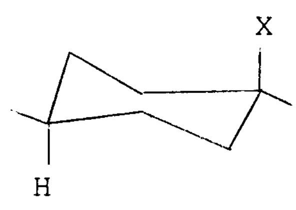

bedeutet.

8. r-1-Cyan-1-pentyl-cis-4-(trans-4-pentylcyclohexyl)-cyclohexan.

9. Verfahren zur Herstellung von Cyclohexanderivaten der Formel I nach Anspruch 1, dadurch gekennzeichnet, daß man eine Verbindung, die sonst der Formel I entspricht, aber anstelle von H-Atomen eine oder mehrere reduzierbare Gruppen und/oder C-C-Bindungen enthält, mit einem reduzierenden Mittel behandelt,

oder daß man an eine Verbindung, die sonst der Formel I entspricht, aber anstelle des Restes

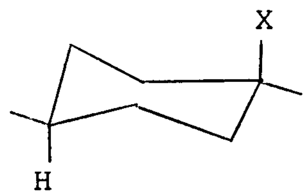

eine 1-Cyclohexen-1,4-diylgruppe enthält, eine Verbindung der Formel HX (worin X, F, Cl, Br oder CN bedeutet) anlagert,

oder daß man zur Herstellung von Estern der Formel I (worin $R^1$ und/oder $R^2$ -O-COR bedeuten und/oder worin $Z^1$ und/oder $Z^2$ -CO-O oder -O-CO- bedeuten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten der Formel I (worin $A^1$ und/oder $A^2$ 1,3-Dioxan-2,5-diyl bedeuten) einen entsprechenden Aldehyd mit einem entsprechenden Diol umsetzt,

oder daß man zur Herstellung von Nitrilen der Formel I (worin $R^1$ und/oder $R^2$ CN bedeuten und/oder X eine CN-Gruppe bedeutet) ein entsprechendes Carbonsäureamid dehydratisiert oder ein entsprechendes Carbonsäurehalogenid mit Sulfamid umsetzt,

oder daß man zur Herstellung von Nitrilen der Formel I (worin X, CN bedeutet; worin ferner einer der Reste $Z^1$ bzw. $Z^2$ eine Einfachbindung bedeutet) ein Nitril der Formel IV

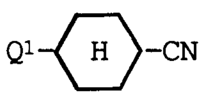

IV

worin
$Q^1$ $R^1$-$(A^1)_m Z^1$-
bedeutet und
$R^1$, $A^1$, $Z^1$ und m die angegebenen Bedeutungen haben,
mit einer Verbindung der Formel V

$Q^2$-$X^1$ V

worin
$Q^2$ $R^2$-$(A^2)_n$-
und $X^1$ Cl, Br, I, OH oder eine reaktionsfähige veresterte OH-Gruppe bedeuten und $R^2$, $A^2$ und n die angegebenen Bedeutungen haben, umsetzt,

oder daß man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ eine $-OCH_2$- oder $-CH_2O$-Gruppe ist) eine entsprechende Hydroxyverbindung verethert,

oder daß man zur Herstellung von Verbindungen der Formel I, die $CF_3$-Gruppen enthalten, eine entsprechende Carbonsäure mit $SF_4$ umsetzt,

und/oder daß man gegebenenfalls eine Chlor- oder Bromverbindung der Formel I (worin $R^1$ und/oder $R^2$ Cl oder Br bedeuten und/oder worin A durch mindestens ein Chlor- oder Bromatom substituiert ist) mit einem Cyanid umsetzt,

oder daß man gegebenenfalls eine Verbindung der Formel I aus einem ihrer Säureadditionssalze durch Behandeln mit einer Base freisetzt.

**10.** Verfahren zur Herstellung von Verbindungen der Formeln If, In, Ir, Iaj,

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

$R^1$-Cy-A-$R^2$ Iaj

worin

$R^1$, $R^2$, $Z^1$, $Z^2$ und Cy die in Anspruch 1 angegebenen Bedeutungen haben und

A 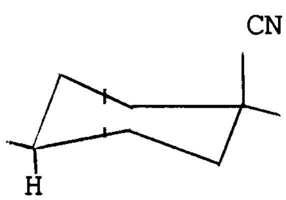

bedeutet,

dadurch gekennzeichnet, daß man an Verbindungen, die sonst der Formel I entsprechen, aber anstelle des Restes wo A jetzt eine Cyclohexen-1,4-diylgruppe enthält, eine Verbindung der Formel HCN addiert.

**11.** Cyclohexenderivate der Formel If, In, Ir, Iaj,

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

wobei
A Cyclohexen-1,4-diyl
bedeutet.

**12.** 1-Pentyl-4-(trans-4-pentylcyclohexyl)-cyclohexen.

**13.** Verwendung der Verbindungen der Formel I nach Anspruch 1 als Komponenten flüssigkristalliner Dielektrika für elektrooptische Anzeigeelemente.

**14.** Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

**15.** Elektrooptische Anzeigeelemente, dadurch gekennzeichnet, daß es ein Dielektrikum nach Anspruch 11 enthält.

**Claims**

**1.** Cyclohexane derivatives of the formula I

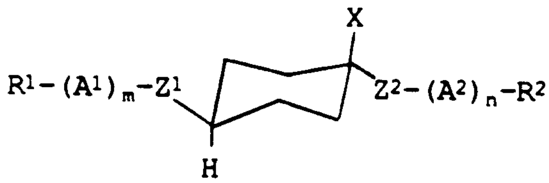

I

wherein $R^1$ and $R^2$ are each straight-chain or at most single-branch alkyl groups having 1-10 C atoms, in which, in addition, one or two $CH_2$ groups can also be replaced by O atoms, F, Cl, Br, CN or -O-COR,$A^1$ and $A^2$ are each 1,4-phenylene, 1,4-cyclohexylene, 1,3-dioxane-2,5-diyl, piperidine-1,4-diyl, 1,4-bicyclo(2,2,2)octylene or pyrimidine-2,5-diyl groups which are unsubstituted or substituted by 1 - 4 F atoms, X is alkyl, alkoxy, fluorinated alkyl or fluorinated alkoxy, each of which has 1-5 C atoms, F, Cl, Br or CN, $Z^1$ and $Z^2$ are each -CO-O-, -O-CO-, $-CH_2CH_2-$, $-OCH_2-$, $-CH_2O-$ or a single bond,R is an all group which has 1 - 5 C atoms, m is 1 or 2 and n is 0 or 1, it being possible, where m = 2, for the two groups $A^1$ to be identical or different from one another and for the groups $R^1$ $-(A^1)_m-Z^1$ and $-Z^2-(A^2)_n-R^2$ to be in the trans position relative to one another, while the substituent X is in the cis position relative to the opposite group.

**2.** Cyclohexane derivatives according to Claim 1 characterised in that X is a CN, $CH_3$, $CH_3O$ or $CF_3$ group.

**3.** Cyclohexane derivatives according to Claim 1 or 2, characterised in that $A^1$ and $A^2$ are each 1,4-cyclohexylene, or 1,4-phenylene which is unsubstituted or substituted by 1-4 fluorine atoms.

**4.** Cyclohexane derivatives according to at least one of Claims 1 to 3, characterised in that $Z^1$ and $Z^2$ are single bonds.

**5.** Cyclohexane derivatives according to at least one of Claims 1 to 4, characterised in that n is 0.

**6.** Cyclohexane derivatives according to at least one of Claims 1 to 5, characterised in that $R^1$ and $R^2$ are ethyl, propyl, butyl, pentyl, hexyl, heptyl, octyl, nonyl, decyl, ethoxy, propoxy, butoxy, pentoxy, hexoxy, heptoxy, octoxy, nonoxy, decoxy, 2-oxapropyl (= methoxymethyl), 2- (=ethoxmethyl) or 3-oxabutyl (=2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, 2-, 3-, 4-, 5-, 6-, 7-, 8- or 9-oxadecyl, 1,3-dioxabutyl (=methoxymethoxy), 1,3-, 1,4- or 2,4- dioxapentyl, 1,3-, 1,4- 1,5-, 2,4-, 2,5- or 3,5-dioxahexyl, 1,3-, 1,4- 1,5-, 1,6- 2,4-, 2,5-, 2,6-, 3,5-, 3,6- or 4,6- dioxaheptyl, isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, isopropoxy, 2-methylpropoxy, 2-methylbutoxy, 3-methylbutoxy, 2-methylpentoxy, 3-methylpentoxy, 2-ethylhexoxy, 1-methylhexoxy, 1-methylheptoxy, 2-oxa-3-methylbutyl or 3-oxa-4-methylpentyl.

**7.** Cyclohexane derivatives according to at least one of Claims 1 to 6, characterised in that they conform to the formulae Iaj or Iat,

$R^1$-Cy-A-$R^2$ Iaj

$R^1$-Phe-Phe-A-$R^2$ Iat

wherein Cy is 1,4-cyclohexylene, Phe is 1,4-phenylene which is unsubstituted or substituted by 1-4 fluorine atoms and A is

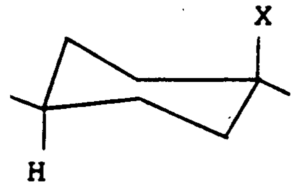

8. r-1-Cyano-1-pentyl-cis-4-(trans-4-pentylcyclohexyl)-cyclohexane.

9. Process for the preparation of cyclohexane derivatives of the formula I according to Claim 1, characterised in that a compound which otherwise corresponds to the formula I, but contains one or more reducible groups and/or C-C bonds instead of H atoms is treated with a reducing agent, or in that a compound of the formula HX (wherein X is F, Cl, Br or CN) is added onto a compound which otherwise corresponds to the formula I, but contains, instead of the radical, a 1-cyclohexene-1,4-diyl group

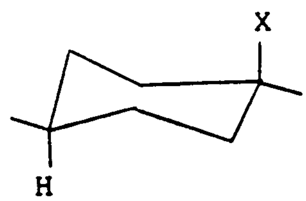

or in that in order to prepare esters of the formula I (wherein $R^1$ and /or $R^2$ are -O-COR and/or wherein $Z^1$ and/or $Z^2$ are -CO-O- or -O-CO-), a corresponding carboxylic acid or one of its reactive derivatives is reacted with a corresponding alcohol or one of its reactive derivatives, or in that in order to prepare dioxane derivatives of the formula I (wherein $A^1$ and/or $A^2$ are 1,3-dioxane-2,5-diyl), a corresponding aldehyde is reacted with a corresponding diol, or in that in order to prepare nitriles of the formula I (wherein $R^1$ and/or $R^2$ are CN and/or wherein X is a CN group), a corresponding carboxamide is dehydrated or a corresponding carboxylic acid halide is reacted with sulfamide, or in that in order to prepare nitriles of the formula I (wherein X is CN; furthermore wherein one of the radicals $Z^1$ and $Z^2$ is a single bond), a nitrile of the formula IV

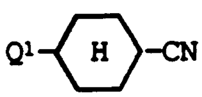

IV

wherein $Q^1$ is $R^1$-$(A^1)_m$-$Z^1$- and $R^1$, $A^1$, $Z^1$ and m have the meanings indicated, is reacted with a compound of the formula V

$Q^2$-$X^1$ V

wherein $Q^2$ is $R^2$-$(A^2)_n$- and $X^1$ is Cl, Br, I, OH or a reactively esterified OH group, and $R^2$, $A^2$ and n have the meanings indicated, or in that in order to prepare ethers of the formula I (wherein $R^1$ and/or $R^2$ are an alkyl group in which one or two $CH_2$ groups are replaced by O atoms and/or $Z^1$ and/or $Z^2$ are a $-OCH_2-$ or $-CH_2O-$ group), a corresponding hydroxy compound is etherified, or in that in order to prepare compounds of the formula I containing $CF_3$ groups, a corresponding carboxylic acid is reacted with $SF_4$, and/or in that, if appropriate, a chlorine or bromine compound of the formula I (wherein $R^1$ and/or $R^2$ are Cl or Br and/or wherein A is substituted by at least one chlorine or bromine atom) is reacted with a cyanide, or in that, if appropriate, a compound of the formula I is liberated from one of its acid addition salts by treatment with a base.

10. Process for the preparation of compounds of the formulae If, In, Ir, Iaj,

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

$R^1$-Cy-A-$R^2$ Iaj

wherein $R^1$, $R^2$, $Z^1$, $Z^2$ and Cy have the meanings indicated in Claim 1, A is

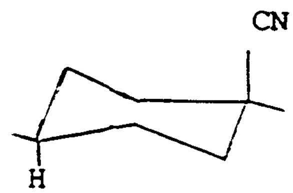

characterised in that a compound of the formula HCN is added onto compounds which otherwise correspond to the formula I, but instead of the radical where A now contains a cyclohexene-1,4-diyl group [sic].

**11.** Cyclohexene derivative of the formula If, In, Ir and Iaj [sic]

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

where
A is cyclohexene-1,4-diyl.

**12.** 1-pentyl-4-(trans-4-pentylcyclohexyl)-cyclohexene.

**13.** The use of the compounds of the formula I according to Claim 1 as components of liquid-crystal dielectrics for electro-optical display elements.

**14.** Liquid-crystal dielectric for electro-optical display elements which has at least two liquid-crystal components, characterised in that at least one component is a compound of the formula I according to Claim 1.

**15.** Electro-optical display element characterised in that it contains a dielectric according to Claim 11.

## Revendications

**1.** Dérivés de cyclohexane de formule I

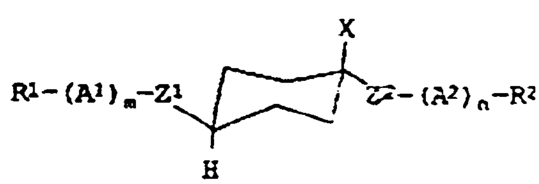

où

$R^1$ et $R^2$ représentent chacun des groupes alkyle en chaîne droite ou portant au plus une ramification et ayant 1-10 atomes de carbone, dans lesquels également un ou deux groupes $CH_2$ peuvent être remplacés par des atomes d'oxygène, F, Cl, Br, CN ou -O-COR,

$A^1$ et $A^2$ représentent chacun des groupes 1,4-phénylène, 1,4-cyclohexylène, 1,3-dioxanne-2,5-diyle, pipéridine-1,4-diyle, 1,4-bicyclo(2,2,2)-octylène ou pyrimidine-2,5-diyle, non-substitués ou substitués par 1-4 atomes de fluor,

X est un radical alkyle, alcoxy, alkyle fluoré ou alcoxy fluoré ayant 1-5 atomes de carbone, F, Cl, Br ou CN,

$Z^1$ et $Z^2$ représentent chacun -CO-O-, -O-CO-, $-CH_2-CH_2-$, $-OCH_2-$, $-CH_2O-$ ou une liaison simple,

R est un groupe alkyle ayant 1-5 atomes de carbone,

m est 1 ou 2 et

n est 0 ou 1,

tandis que pour m = 2 les deux groupes $A^1$ peuvent être identiques ou différents l'un de l'autre, et les groupes $R^1-(A^1)_m-Z^1-$ et $-Z^2-(A^2)_n-R^2$ sont en position trans l'un par rapport à l'autre, alors que le substituant X est en position cis par rapport au groupe opposé.

**2.** Dérivés de cyclohexane selon la revendication 1, caractérisés en ce que X désigne un groupe CN-, $CH_3$-, $CH_3O$- ou $CF_3$-.

**3.** Dérivés de cyclohexane selon l'une des revendications 1 ou 2, caractérisés en ce que $A^1$ et $A^2$ représentent chacun un groupe 1,4-cyclohexylène ou 1,4-phénylène substitué par 1-4 atomes de fluor.

**4.** Dérivés de cyclohexane selon au moins l'une des revendications 1 à 3, caractérisés en ce que $Z^1$ et $Z^2$ représentent des liaisons simples.

**5.** Dérivés de cyclohexane selon au moins l'une des revendications 1 à 4, caractérisés en ce que n est 0.

**6.** Dérivés de cyclohexane selon au moins l'une des revendications 1 à 5, caractérisés en ce que $R^1$ et $R^2$ représentent des groupes éthyle, propyle, butyle, pentyle, hexyle, heptyle, octyle, nonyle, décyle, éthoxy, propoxy, butoxy, pentoxy, hexoxy, heptoxy, octoxy, nonoxy, décoxy, 2-oxapropyle,(= méthoxyméthyle), 2- (= éthoxyméthyle) ou 3-oxabutyle (= 2-méthoxyéthyle), 2-, 3- ou 4-oxapentyle, 2-, 3-, 4- ou 5-oxahexyle, 2-, 3-, 4-, 5- ou 6-oxapheptyle, 2-, 3-, 4-, 5-, 6- ou 7-oxaoctyle, 2-, 3-, 4-, 5-, 6-, 7- ou 8-oxanonyle, 2-, 3-, 4-, 5-, 6-, 7-, 8- ou 9-oxadécyle, 1,3-dioxabutyle (= méthoxyméthoxy), 1,3-, 1,4- ou 2,4-dioxapentyle, 1,3-, 1,4-, 1,5-, 2,4-, 2,5- ou 3,5-dioxahexyle, 1,3-, 1,4-, 1,5-, 1,6-, 2,4-, 2,5-, 2,6-, 3,5-, 3,6- ou 4,6-dioxaheptyle, isopropyle, 2-butyle (= 1-méthylpropyle), isobutyle (= 2-méthylpropyle), 2-méthylbutyle, isopentyle (= 3-méthylbutyle), 2-méthylpentyle, 3-méthylpentyle, 2-éthylhexyle, 2-propylpentyle, isopropoxy, 2-méthylpropoxy, 2-méthylbutoxy, 3-méthylbutoxy, 2-méthylpentoxy, 3-méthylpentoxy, 2-éthylhexoxy, 1-méthylhexoxy, 1-méthylheptoxy, 2-oxa-3-méthylbutyle ou 3-oxa-4-méthylpentyle.

**7.** Dérivés de cyclohexane selon au moins l'une des revendications 1 à 6, caractérisés en ce qu'ils correspondent aux formules Iaj ou Iat,

$R^1$-Cy-A-$R^2$ Iaj

$R^1$-Phe-Phe-A-$R^2$ Iat

où Cy représente un radical 1,4-cyclohexylène, Phe un radical 1,4-phénylène non-substitué ou substitué par 1-4 atomes de fluor, et A un radical

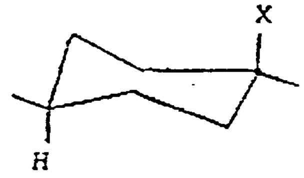

**8.** r-1-cyano-1-pentyl-cis-4-(trans-4-pentylcyclohexyl)-cyclohexane.

**9.** Procédé pour la production de dérivés de cyclohexane de formule I selon la revendication 1, caractérisé en ce qu'on traite avec un moyen réducteur un composé qui correspond par ailleurs à la formule I, mais contient à la place d'atomes d'hydrogène un ou plusieurs groupes réductibles et/ou des liaisons C-C,

ou qu'on fixe par addition sur un composé qui par ailleurs correspond à la formule I, mais contient à la place du reste

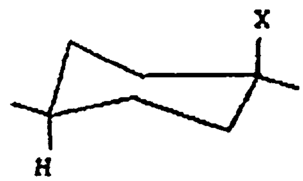

un groupe 1-cyclohexène-1,4-diyle, un composé de formule HX (où X désigne F, Cl, Br ou CN),

ou que pour la production d'esters de formule I (où $R^1$ et/ou $R^2$ sont -O-COR et/ou dans lesquels $Z^1$ et/ou $Z^2$ représentent -CO-O- ou -O-CO-) on fait réagir un acide carboxylique correspondant ou l'un de ses dérivés réactifs avec un alcool correspondant ou avec l'un de ses dérivés réactifs,

ou que pour la production de dérivés de dioxanne de formule I (où $A^1$ et/ou $A^2$ sont un radical 1,3-dioxanne-2,5-diyle) on fait réagir un aldéhyde correspondant avec un diol correspondant,

ou que pour la production de nitriles de formule I (où $R^1$ et/ou $R^2$ sont CN et/ou X représente un groupe CN) on déshydrate un amide d'acide carboxylique correspondant ou on fait réagir un halogénure d'acide carboxylique avec un sulfamide,

ou que pour la production de nitriles de formule I (où X est CN, tandis qu'en outre l'un des restes $Z^1$ ou $Z^2$ respectivement représente une liaison simple) on fait réagir un nitrile de formule IV

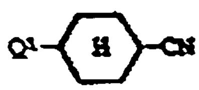

IV

où
$Q^1$ est $R^1$-$(A^1)_m$$Z^1$-
et
$R^1$, $A^1$, $Z^1$ et m ont les significations indiquées,
avec un composé de formule V

$Q^2$-$X^1$ V

où
$Q^2$ est $R^2$-$(A^2)_n$-
et $X^1$ représente Cl, Br, I, OH ou un groupe OH estérifié réactif et $R^2$, $A^2$ et n ont les significations indiquées,

ou que pour la production d'éthers de formule I (où $R^1$ et/ou $R^2$ représente un groupe alkyle, tandis qu'un ou deux groupes $CH_2$ sont remplacés par des atomes d'oxygène et/ou $Z^1$ et/ou $Z^2$ est un groupe $-OCH_2-$ ou $-CH_2O-$) on éthérifie un composé hydroxylé correspondant,

ou que pour la production de composés de formule I qui contiennent des groupes $CF_3$- on fait réagir un acide carboxylique correspondant avec $SF_4$,

et/ou qu'éventuellement on fait réagir un composé chloré ou bromé de formule I (où $R^1$ et/ou $R^2$ sont Cl ou Br et/ou dans lequel A est substitué par au moins un atome de chlore ou de brome) avec un cyanure,

ou qu'éventuellement on libère un composé de formule I à partir d'un de ses sels d'addition avec un acide par traitement avec une base.

**10.** Procédé pour la production de composés de formules If, In, Ir, Iaj,

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

$R^1$-Cy-A-$R^2$ Iaj

où
$R^1$, $R^2$, $Z^1$, $Z^2$ et Cy ont la signification indiquée dans la revendication 1 et
A est un radical

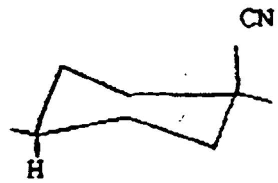

caractérisé en ce qu'on fixe par addition à des composés qui correspondent par ailleurs à la formule I, mais contiennent à la place du reste A un groupe cyclohexène-1,4-diyle, un composé de formule HCN.

**11.** Dérivés de cyclohexane de formules If, In, Ir,

$R^1$-Cy-$Z^1$-A-$Z^2$-$R^2$ If

$R^1$-Cy-$Z^1$-A-$Z^2$-Cy-$R^2$ In

$R^1$-Cy-Cy-$Z^1$-A-$Z^2$-$R^2$ Ir

où
A désigne un radical cyclohexène-1,4-diyle.

**12.** 1-pentyl-4-(trans-4-pentylcyclohexyl)-cyclohexène.

**13.** Utilisation des composés de formule I selon la revendication 1 comme constituants de diélectriques à cristaux liquides pour éléments d'affichage électro-optiques.

**14.** Diélectrique à cristaux liquides pour éléments d'affichage électro-optiques ayant au moins deux constituants à cristaux liquides, caractérisé en ce qu'au moins un constituant est un composé de formule I selon la revendication 1.

**15.** Elément d'affichage électro-optique, caractérisé en ce qu'il contient un diélectrique selon la revendication 11.